# EUROPEAN PATENT SPECIFICATION

(11) **EP 2 841 064 B1**
(45) Date of publication and mention of the grant of the patent: **12.06.2019**
(21) Application number: 13782164.1
(22) Date of filing: 26.04.2013
(51) Int. Cl.: A61K 31/16, A61K 9/08, A61P 27/12

(54) **USE OF N-ACETYLCYSTEINE AMIDE IN THE TREATMENT OF CATARACTS**
VERWENDUNG VON N-ACETYLCYSTEINAMID BEI DER BEHANDLUNG VON KATARAKTEN
UTILISATION DE N-ACÉTYLCYSTÉINE AMIDE DANS LE TRAITEMENT DE LA CATARACTE

(30) Priority: 26.04.2012 US 201261638799 P
(43) Date of publication of application: 04.03.2015
(73) Proprietor: Sentient Lifesciences, Inc., New York, NY 10022 (US)
(72) Inventor: GOLDSTEIN, Glenn, A., New York, NY 10022 (US)
(74) Representative: Harris, Jennifer Lucy
(86) International application number: PCT/US2013/038415
(87) International publication number: WO 2013/163545

(56) References cited:
- WO-A2-2006/116353
- US-A- 5 519 054
- US-A1- 2010 137 441
- ZHANG SHU ET AL: "Effects of N-acetylcysteine and glutathione ethyl ester drops on streptozotocin-induced diabetic cataract in rats", MOLECULAR VISION, vol. 14, no. 103, May 2008 (2008-05), pages 862-870, XP002747514, ISSN: 1090-0535
- CAREY, J. W. ET AL.: 'In vivo inhibition of 1-buthionine-(S,R)-sulfoximine- induced cataracts by a novel antioxidant, N-acetylcysteine amide' FREE RADICAL BIOLOGY & MEDICINE vol. 50, 2011, pages 722 - 729, XP028363108
- STANZIALE, S. F. ET AL.: 'Infection with oncolytic herpes simplex virus-1 induces apoptosis in neighboring human cancer cells: a potential target to increase anticancer activity' CLINICAL CANCER RESEARCH vol. 10, no. 9, 2004, pages 3225 - 3232, XP055175006
- SCHIMEL, A. M. ET AL.: 'N-acetylcysteine amide (NACA) prevents retinal degeneration by up-regulating reduced glutathione production and reversing lipid peroxidation' THE AMERICAN JOURNAL OF PATHOLOGY vol. 178, no. 5, 2011, pages 2032 - 2043, XP055043895
- CAREY, J. W. ET AL.: 'N-acetyl-L-cysteine amide protects retinal pigment epithelium against methamphetamine-induced oxidative stress' JOURNAL OF BIOPHYSICAL CHEMISTRY vol. 3, no. 2, 29 May 2012, pages 101 - 110, XP055175007

## Description

### BACKGROUND

Oxidative stress plays an important role in the progression of various diseases, causing damage to proteins, DNA, and lipids. Low molecular weight, hydrophobic antioxidant compounds are useful in treating conditions of peripheral tissues. A deficiency of cellular antioxidants may lead to excess free radicals, which cause macromolecular breakdown, lipid peroxidation, buildup of toxins and ultimately cell death. Because of the importance of antioxidant compounds in preventing this cellular oxidation, natural antioxidants, such as glutathione (GSH) (γ-glutamyl cysteinyl glycine) are continuously supplied to the tissues. GSH is synthesized by most cells and is one of the primary cellular antioxidants responsible for maintaining the proper oxidation state within the body. When oxidized, GSH forms a dimer, GSSG, which may be recycled in organs producing glutathione reductase. In human adults, reduced GSH is produced from GSSG, primarily in the liver, and to a smaller extent, by skeletal muscle and red and white blood cells, and is distributed through the blood stream to other tissues in the body.

However, under certain conditions, the normal, physiologic supplies of GSH are insufficient, its distribution is inadequate or local oxidative demands are too high to prevent cellular oxidation. Under other conditions, the production of and demand for cell antioxidants, such as GSH, are mismatched, thus leading to insufficient levels of these molecules in the body. In other cases, certain tissues or biological processes consume the antioxidants so that their intracellular levels are suppressed. In either case, increased serum levels of antioxidant, e.g., glutathione, leads to increased amounts of the antioxidant that can be directed into cells. In facilitated transport systems for cellular uptake, the concentration gradient that drives uptake is increased.

Glutathione N-acetylcysteine amide (NAC amide), the amide form of N-acetylcysteine (NAC), is a low molecular weight thiol antioxidant and a Cu²⁺ chelator. NAC amide provides protective effects against cell damage. NAC amide was shown to inhibit tert-butylhydroxyperoxide (BuOOH)-induced intracellular oxidation in red blood cells (RBCs) and to retard BuOOH-induced thiol depletion and hemoglobin oxidation in the RBCs. This restoration of thiol-depleted RBCs by externally applied NAC amide was significantly greater than that found using NAC. Unlike NAC, NAC amide protected hemoglobin from oxidation. (L. Grinberg et al., Free Radic Biol Med., 2005 Jan. 1, 38(1):136-45). In a cell-free system, NAC amide was shown to react with oxidized glutathione (GSSG) to generate reduced glutathione (GSH). NAC amide readily permeates cell membranes, replenishes intracellular GSH, and, by incorporating into the cell's redox machinery, protects the cell from oxidation. Because of its neutral carboxyl group, NAC amide possesses enhanced properties of lipophilicity and cell permeability. (See, e.g., U.S. Pat. No. 5,874,468 to D. Atlas et al.). NAC amide is also superior to NAC and GSH in crossing the cell membrane, as well as the blood-brain barrier.

NAC amide may function directly or indirectly in many important biological phenomena, including the synthesis of proteins and DNA, transport, enzyme activity, metabolism, and protection of cells from free-radical mediated damage. NAC amide is a potent cellular antioxidant responsible for maintaining the proper oxidation state within the body. NAC amide can recycle oxidized biomolecules back to their active reduced forms and may be as effective, if not more effective, than GSH as an antioxidant.

J. W. Carey et al., Free Radical Biology & Medicine, 50 (2011), 722-729 discloses effects of NAC amide on cataract development.

There is a need in the art for other compounds and therapeutic aspects to treat a number of diseases that are linked to oxidative stress and the presence of free oxygen radicals and associated disease pathogenesis in cells and tissues. Needed are antioxidant compounds, other than GSH, that are safe and even more potent, to overcome high oxidative stress in the pathogenesis of diseases. Ideally, such compounds should readily cross the blood-brain barrier and easily permeate the cell membrane. Antioxidants such as vitamins E and C are not completely effective at decreasing oxidative stress, particularly because, in the case of vitamin E, they do not effectively cross through the cell membrane to reach the cytoplasm so as to provide antioxidant effects.

### SUMMARY

The invention is defined in the appended claims. The invention provides a composition comprising a therapeutically effective amount of N-acetylcysteine amide (NAC amide) for use in a method of treating cataracts in a subject (e.g. human), wherein the treatment of the cataract comprises reduction of a grade II cataract to a grade I cataract, reduction of a grade III cataract to a grade II or reduction of a grade III cataract to a grade I cataract wherein the grade is made according to Lens Opacity Classification System III or the treatment of cataract comprises reduction of the cataract so there is no sign of cataract in the subject after treatment.

In certain embodiments, the composition also includes a pharmaceutically acceptable salt or excipient.

In other embodiments, the NACA is administered systemically. Optionally, NACA can administered intraperitoneally or intravenously. In another embodiment, the NACA is administered directly onto or into the eye of the subject. The NACA can be administered intraocularly.

In certain embodiments, the subject is a mammal. Optionally, the mammal is a human.

In other embodiments, the cataract can be selected from the group consisting of nuclear sclerosis, cortical cataract and posterior subcapsular cataract.

In other embodiments, the NACA is administered at between 5 and 10,000 mg/kg.

The disclosure also provides a method of treating or preventing the formation or induction of cataracts in a subject (e.g. human) comprising administering N-acetylcysteine amide (NAC amide), or a pharmaceutically acceptable salt, ester, or derivative thereof, in a dose effective for treating or preventing the formation or induction of cataracts.

The disclosure also provides a method of treating retinal pigment epithelial dysfunction or death in degenerative retinal diseases, including age-related macular degeneration, comprising administering N-acetylcysteine amide (NAC amide), or a pharmaceutically acceptable salt, ester, or derivative thereof.

The disclosure (not within the scope of the claims) also provides a method of treating or preventing age-related macular degeneration in a subject (e.g. human) comprising administering N-acetylcysteine amide (NAC amide), or a pharmaceutically acceptable salt, ester, or derivative thereof, in a dose effective for treating or preventing age-related macular degeneration.

The disclosure (not within the scope of the claims) also provides a method of treating or preventing dry macular degeneration in a subject (e.g. human) comprising administering N-acetylcysteine amide (NAC amide), or a pharmaceutically acceptable salt, ester, or derivative thereof, in a dose effective for treating or preventing dry macular degeneration.

The disclosure (not within the scope of the claims) also provides a method of treating spinal cord injury in a subject (e.g. human) comprising administering N-acetylcysteine amide (NAC amide), or a pharmaceutically acceptable salt, ester, or derivative thereof, in a dose effective for treating spinal cord injury. In some embodiment, the spinal cord injury is cause by a traumatic force or blunt trauma.

The disclosure (not within the scope of the claims) also provides a method of treating contrast induced nephropathy and/or reperfusion injury in a subject (e.g. human) comprising administering N-acetylcysteine amide (NAC amide), or a pharmaceutically acceptable salt, ester, or derivative thereof, in a dose effective for treating Contrast induced nephropathy and/or reperfusion injury.

The dose for administration is 50-10,000 mg per dose, or in an equivalent amount. In some embodiments, the dose for administration is 25-500 mg per dose, or in an equivalent amount. In some embodiments, the NAC amide is delivered orally via a capsule.

In some embodiments, the dose is at least 300mg/kg where there is near complete protection of mitochondrial function.

The disclosure also provides a dosage form for administration to the eye comprising NACA. In certain embodiments, the dosage form is eye drops.

In other embodiments, the dosage form comprises between 5 and 10,000 mg of NACA.

The disclosure (not within the scope of the claims) also provides a method of treating neuronal trauma in a subject in need thereof comprising administering to the subject a composition comprising a therapeutically effective dose of NACA. In certain embodiments, the neuronal trauma is spinal trauma. In other embodiments, the NACA is administered systemically. Optionally, the NACA is administered intraperitoneally or intravenously.

In other embodiments, the NACA is administered directly to the neuronal trauma. Optionally, the neuronal trauma is spinal.

In other embodiments, the NACA is administered at between 75 and 600 mg/kg or at between 200 and 400 mg/kg to the subject.

The disclosure (not within the scope of the claims) also provides a method of increasing respiratory controlled ratio (RCR) and/or respiration rate in spinal cord tissue comprising administering to the spinal cord tissue a composition comprising an effective amount of NACA. In certain embodiments, the injury is a result of trauma.

In other embodiments, the spinal cord tissue includes synaptic cells. In one aspect of this embodiment, the RCR and/or respiration rate is increased in the synaptic cells.

In other embodiments, the spinal cord tissue includes non-synaptic cells. In one aspect of this embodiment, the RCR and/or respiration rate is increased in the non-synaptic cells. In certain aspects of this embodiment, the non-synaptic cells comprise neuronal soma cells. In other aspects of this embodiment, the non-synaptic cells comprise glial cells.

In other embodiments, the NACA is administered at between 75 and 600 mg/kg or at between 200 and 400 mg/kg to the subject.

In other embodiments, the subject is a mammal. Optionally, the mammal is a human.

The disclosure (not within the scope of the claims) also provides a method of treating acquired immune deficiency syndrome (AIDS) in a subject in need thereof comprising administering to the subject a composition comprising a therapeutically effective amount of NACA. In some embodiments, the composition further comprises a pharmaceutically acceptable salt or excipient.

In other embodiments, the NACA is administered systemically. Optionally, the NACA is administered intraperitoneally or intravenously.

In other embodiments, the NACA is administered at between 5 and 10,000 mg/kg.

In yet other embodiments, the subject is a mammal. Optionally, the mammal is a human.

In certain embodiments, the treatment reduces the reverse transcription activity in cells infected by HIV in the subject. In other embodiments, the treatment reduces immunodeficiency in the subject.

The disclosure (not within the scope of the claims) also provides a method of reducing human immunodeficiency virus (HIV) replication in a mammalian cell comprising administering to the cell a compound comprising an effective amount of NACA. In certain embodiments, the cells are peripheral blood mononuclear cells. In certain aspects of this embodiment, the peripheral blood mononuclear cells include a cell type selected from lymphocytes, monocytes and macrophages. Optionally, peripheral blood mononuclear cells comprise lymphocytes. The lymphocytes can include T-cell lymphocytes.

In other embodiments, the reverse transcriptase activity in the peripheral blood mononuclear cells is decreased. In certain aspects of this embodiment, the peripheral blood mononuclear cells is decreased by at least 50% or at least 90%.

In other embodiments, the mammalian cells are human cells.

The disclosure (not within the scope of the claims) also provides a composition comprising NACA and an oncolytic virus. In certain embodiments, the composition also includes a pharmaceutically acceptable salt or excipient.

In other embodiments, the oncolytic virus is transformed into a carrier cell. In certain aspects of this embodiment, the carrier cell is a neural stem cell. Optionally, the neural stem cell is an immortalized cell line. The immortalized cell line can include *v-myc*. In certain specific embodiments, the cell line is HB1.F3.CD. In other specific embodiments, the oncolytic virus is CRAd-S-pk7.

The disclosure (not within the scope of the claims) also provides a method of treating cancer in a subject in need thereof comprising administering a composition comprising NACA and the oncolytic virus as described in any of the embodiments above. In certain embodiments, the NACA is administered systemically. According to certain aspects of these embodiments, the NACA is administered intraperitoneally or intravenously.

In other embodiments, the NACA is administered at between 100 and 400 mg/kg.

In other embodiments, the cancer is selected from the group consisting of glioma, breast cancer, lung cancer, brain cancer, melanoma, prostate cancer, ovarian cancer, pancreatic cancer, liver cancer, colon cancer, cervical cancer, bladder cancer, spleen cancer, head and neck cancer, or bone cancer. In certain embodiments, the cancer is glioma.

In other embodiments, the subject is a mammal. Optionally, the mammal is a human.

The disclosure (not within the scope of the claims) also provides a method of improving the viability of carrier cells comprising oncolytic virus in a mammalian subject comprising co-administering the carrier cells comprising the oncolytic virus as described in any of the embodiments above, with an effective amount of NACA. In certain embodiments, the NACA is administered systemically. In certain aspects of these embodiments, the NACA is administered intraperitoneally or intravenously.

In other embodiments, the NACA is administered at between 100 and 400 mg/kg.

In other embodiments, the subject is a mammal. Optionally, the mammal is a human.

The disclosure provides a method of inducing cancer cell oncolysis comprising administering to the cancer cell the oncolytic virus as described in any of the embodiments above and NACA. In certain embodiments, the NACA is administered systemically. In certain aspects of these embodiments, the NACA is administered intraperitoneally or intravenously.

In other embodiments, the NACA is administered at between 5 and 10,000 mg/kg.

In other embodiments, the cancer is selected from the group consisting of glioma, breast cancer, lung cancer, brain cancer, melanoma, prostate cancer, ovarian cancer, pancreatic cancer, liver cancer, colon cancer, cervical cancer, bladder cancer, spleen cancer, head and neck cancer, or bone cancer. In certain embodiments, the cancer is glioma.

The disclosure (not within the scope of the claims) also provides a method of improving stem cell viability comprising administering an effective amount of NACA to the stem cells. In certain embodiments, the stem cells are neural stem cells.

In other embodiments, the stem cells are present in a subject. The NACA can be administered to the subject. In certain embodiments, the NACA is administered systemically. Optionally, the NACA is administered intraperitoneally or intravenously.

In other embodiments, the NACA is administered at between 5 and 10,000 mg/kg.

### BRIEF DESCRIPTION OF THE DRAWINGS

Figure 1 is a bar graph showing NACA (DP1) post-injury treatment improves mitochondrial function as indicated by the respiratory control ratio (measurement of mitochondrial coupling).
Figure 2 is a bar graph showing that NACA (DP1) post-injury treatment improves mitochondrial function by restoring state III (ATP production) and state V (maximum electron transport) respiration rates.
Figure 3. is a bar graph showing that after 6 weeks of testing, treatment with NACA (DP1) rendered significant increases in the BB scores (∼11-walking) compared to vehicle treatment (∼8-dragging limbs).
Figure 4 is a bar graph showing synaptic mitochondrial oxygen consumption results for from left to right of negative control, vehicle, and 75, 100, 300 and 600 mg of NACA.
Figure 5 is a bar graph showing non-synaptic mitochondrial oxygen consumption results for from left to right of negative control, vehicle, and 75, 100, 300 and 600 mg of NACA.
Figure 6 is a line graph showing improved hindlimb locomotor function beginning 7 days-post SCI.
Figure 7 is a line graph showing amount of reverse transcriptase (RT) activity in non-activated HIV infected U1 cells with varying amounts of NACA.
Figure 8 is a bar graph showing thymidine incorporation in HIV U1 cells in media (RPMI), and activated with TNF-α or IL-6 with varying amounts of NACA.
Figure 9 shows bar graphs showing reverse transcriptase (RT) activity in U1 cells infected with HIV and activated with TNF-α or IL-6 with varying amounts of NACA.
Figure 10 is a bar graph showing vaibility of HIV infected peripheral blood mononuclear cells (PBMCs) in the presence of varying amounts of NACA.
Figure 11 is a bar graph showing amount of HIV RNA produced in HIV infected peripheral blood mononuclear cells (PBMCs) in the presence of varying amounts of NACA.
Figure 12 is a bar graph that shows percent inhibition of HIV replication in HIV infected peripheral blood mononuclear cells (PBMCs) in the presence of varying amounts of NACA.
Figure 13 is a bar graph that shows reverse transcriptase (RT) activity in HIV infected U1 cells activated with TNF-α with varying amounts of NACA.
Figure 14 is a bar graph that shows reverse transcriptase (RT) activity in HIV infected U1 cells activated with IL-6 with varying amounts of NACA.
Figure 15 is a bar graph that shows reverse transcriptase (RT) activity in HIV infected U1 cells activated with PMAwith varying amounts of NACA.
Figure 16 is a schematic that shows a potential mechanism of NACA in inhibition of HIV replication.
Figure 17 is a schematic that shows a potential mechanism of NACA in inhibition of HIV replication.
Figure 18 is a schematic that shows a potential mechanism of NACA in inhibition of HIV replication.
Figure 19A. HB1.F3.CD NSCs were treated with various concentrations of NACA (1, 2.5, 5 and 10 mM), CRAd-S-pk7 (1, 5, 10 and 50 I.U) and the combination of NACA with CRAd-S-pk7. Cell viability was evaluated by MTT assay 72 hours after treatment. Representative digital microscopic image of viable cells are seen in the bottom of Figure 1A. Scale bar, 200 µm.
Figure 19B. HB1.F3.CD cells were infected with CRAd-S-pk7 at 10 and 50 I.U./cell. Media were changed one hour after virus infection and then exposed to 1 mM of NACA or were left untreated. Cell viability was measured by MTT assay at the indicated times.
Figure 19C. The viability of U87 glioma cells after treatment with NACA and CRAd-S-pk7 was determined by MTT assay at 72 hours. The values shown are mean ± SEM. *,P < 0.05, **, P < 0.01 versus control.
Figure 20A. The replication capacity of CRAd-S-pk7 combined with NACA was measured by quantitative RT-PCR. Viral replication was determined by the number of viral E1A copies per ng of DNA from infected NSCs. All samples were analyzed in triplicates. They are presented as mean ± SEM.
Figuire 20B. HB1.F3.CD cells were infected with CRAd-Spk7 in a dose-dependent manner (1, 5, 10 and 50 I.U./cell) and then combined with 1 mM of NACA at 72 hours post treatment.
Figure 20C. The replication of CRAd-S-pk7 treated with 1 mM of NACA was measured by qRT-PCR in a time-dependent manner.
Figure 21A. Media of HB1.F3.CD cells infected with various concentrations of CRAd-S-pk7 (1, 10, 50 and 100 I.U./cell) were collected at 72 hours post treatment and assessed by viral titer assay.
Figure 21B. HB1.F3.CD cells were infected with 50 I.U./cell of CRAd-S-pk7 and infected with 50 I.U./cell of CRAd-S-pk7 and treated with different concentrations of NACA (1, 2.5 and 5 mM). Viral titer was then evaluated.
Figure 21C. HB1.F3.CD cells were infected with several doses of CRAd-S-pk7 and subsequently treated with or without 1 mM of NACA. Three days post-treatment, supernatant was collected and viral titer assay was performed.
Figure 21D. Representative microscopic pictures of viral titer assay. Scale bar, 200 µm.
Figure 21E. HB1.F3.CD cells were infected with CRAd-S-pk7 at 50 I.U./cell with or without 1 mM of NACA. Supernatant (cell free) and the infected cells (cell associated) were separately collected at indicated time-points. Viral titer was then analyzed.
Figure 22A. HB1.F3.CD cells were infected with CRAd-S-pk7 (50 I.U/cell) and then treated with or without 1 mM of NACA. Supernatant was collected four days after initial treatment and used to infect U87 glioma cells. Cell viability was analyzed 4 days later using crystal violet staining (top). Scale bar, 200 µm. Differences in U87 cell viability between CON (control), NACA only, CRAd-S-pk7 only and OV and NACA combination groups were quantified (bottom).
Figure 22B. HB1.F3.CD cells were infected with CRAd-S-pk7 (50 I.U/cell) and then treated with or without 1 mM of NACA. U87-Luc cells were plated in precultured wells containing NSCs loaded with OVs at the following ratio (NSCs: U87-Luc, 1:0.5 and 1:1). NSCs and U87 cells were lysed at 96 hours post co-culture and Luciferase activity was measured. Bar graph shows luciferase activity (arithmetic mean ± SEM) in quadruplicates for each co-culture. *P < 0.05.
Figure 23A. HB1.F3.CD cells were infected with CRAd-S-pk7 (10 and 50 I.U/cell) and then treated with or without 1 mM of NACA for 24 hours. Afterwards, cells were incubated with an oxidant-sensitive fluorogenic reagent, CM-H2DCFDA. Cell image was obtained using a fluorescent microscope. Scale bar, 100 µm. The mean fluorescence of four randomly selected fields was calculated. ROS values were compared with the control group and expressed as the fold of the control level.
Figure 23B. HB1.F3.CD cells were treated with NACA (1, 2.5 and 5 mM) and CRAd-S-pk7 (1, 10 and 50 I.U/cell). After 24 hours, cell lysates were analyzed by immunoblotting.
Figure 23C. HB1.F3.CD cells were treated with combination of NACA and CRAd-S-pk7 as indicated and cells were collected. Cell lysates (30 µg) were analyzed by immunoblotting with p-Akt, p53 and caspase-3 antibodies.
Figure 23D. HB1.F3.CD cells were treated with combination of NACA (1 mM) and CRAd-S-pk7 (50 I.U/cell) and subjected to western blotting. Equal protein loading was verified by anti- β-actin antibody. The quantitative analysis of p-p38 and p-Akt protein levels was determined by densitometry analysis (normalized to actin), represented as a bar graph.
Figure 24A. Kaplan-Meier survival curve of mice implanted with U87 cells that were randomly divided into 4 groups: PBS (n=6), NACA only (250 mg/kg, n=6), HB1.F3.CD cells loaded with CRAd-S-pk7 (50 I.U./cell, n=7) and combination of NACA and HB1.F3.CD cells loaded with CRAd-S-pk7 (n=7). Differences between survival curves were compared using a log-rank test and are shown as P- values. *P < 0.05
Figure 24B. Histological sections of U87 brain tumors were stained with anti-caspase-3 antibody.
Figure 25A. Immunohistochemistry staining showing E1A expression in U87 tumor sections (center and border) of mice treated with PBS, NACA only, HB1.F3.CD cells loaded with NACA and combination of NACA and HB1.F3.CD cells loaded with NACA. Scale bar, 35 µm.
Figure 25B. The right brain hemisphere (the one with implanted tumor cells and intratumoral injection of NSCs) of mice from all four groups (n=4 per group) was collected and in vivo CRAd-S-pk7 replication was quantified by quantitative RT-PCR. Viral progeny was assessed by viral titer assay.

### DETAILED DESCRIPTION

The present invention provides the use of N-acetylcysteine amide (NAC amide or NACA) or derivatives thereof, or a physiologically acceptable derivative, salt, or ester thereof, to treat disorders, conditions, pathologies and diseases that result from, or are associated with, the adverse effects of oxidative stress and/or the production of free radicals in cells, tissues and organs of the body. NACA and its derivatives are provided for use in methods and compositions for improving and treating such disorders, conditions, pathologies and diseases according to the appended claims.

As used herein, a "subject" within the context of the present invention encompasses, without limitation, mammals, e.g., humans, domestic animals and livestock including cats, dogs, cattle and horses. A "subject in need thereof' is a subject having one or more manifestations of disorders, conditions, pathologies, and diseases as disclosed herein in which administration or introduction of NAC amide or its derivatives would be considered beneficial by those of ordinary skill in the art.

In an aspect of the present invention, compositions comprising NAC amide provide an antioxidant to cells and tissues to reduce oxidative stress, and the adverse effects of cellular oxidation, in an organism. The invention provides a pharmaceutically acceptable formulation of NAC amide or derivatives thereof for use in a method of reducing oxidative stress associated with the conditions, diseases, pathologies as described herein, by administering a pharmaceutically acceptable formulation of NAC amide or derivatives thereof to a human or non-human mammal in an amount effective to reduce oxidative stress.

In another aspect of the present invention, NAC amide and its derivatives are provided to treat an organism having a disorder, condition, pathology, or disease that is associated with the overproduction of oxidants and/or oxygen free radical species. According to this invention NAC amide treatment can be prophylactic or therapeutic.

"Therapeutic treatment" or "therapeutic effect" means any improvement in the condition of a subject treated by the methods of the present invention, including obtaining a preventative or prophylactic effect, or any alleviation of the severity of signs or symptoms of a disorder, condition, pathology, or disease or its sequelae, including those caused by other treatment methods (e.g., chemotherapy and radiation therapy), which can be detected by means of physical examination, laboratory, or instrumental methods and considered statistically and/or clinically significant by those skilled in the art.

"Prophylactic treatment" or "prophylactic effect" means prevention of any worsening in the condition of a subject treated by the methods of the present invention, as well as prevention of any exacerbation of the severity of signs and symptoms of a disorder, condition, pathology, or disease or its sequelae, including those caused by other treatment methods (e.g., chemotherapy and radiation therapy), which can be detected by means of physical examination, laboratory, or instrumental methods and considered statistically and/or clinically significant by those skilled in the art.

Also disclosed herein (not within the scope of the claims) is NAC amide used in the treatment and/or prevention of cosmetic conditions and dermatological disorders of the skin, hair, nails, and mucosal surfaces when applied topically. Compositions for topical administration are provided that include (a) NAC amide, or derivatives thereof, or a suitable salt or ester thereof, or a physiologically acceptable composition containing NAC amide or its derivatives; and (b) a topically acceptable vehicle or carrier. Also disclosed herein is a method for the treatment and/or prevention of cosmetic conditions and/or dermatological disorders that entails topical administration of NAC amide- or NAC-amide derivative-containing compositions to an affected area of a patient.

Also disclosed herein is a compound of the formula I: 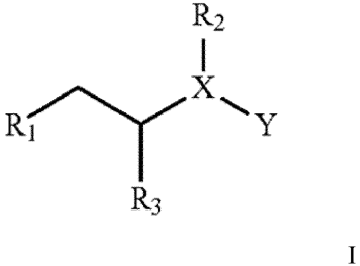 wherein: R₁ is OH, SH, or S--S--Z; X is C or N; Y is NH₂, OH, CH₃-C=O, or NH-CH₃; R.sub.2 is absent, H, or =O R₃ is absent or 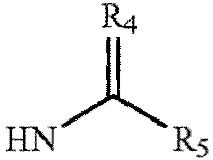 wherein: R₄ is NH or O; R₅ is CF₃, NH₂, or CH₃
and wherein: Z is 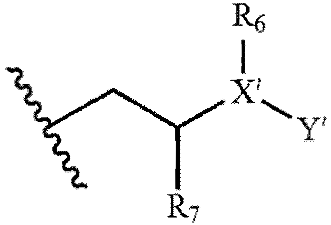 with the proviso that if R₁ is S--S--Z, X and X' are the same, Y and Y' are the same, R₂ and R₆ are the same, and R₃ and R₇ are the same.

Also disclosed herein is a NAC amide compound and NAC amide derivatives comprising the compounds disclosed herein. Other derivatives are disclosed in U.S. Patent No. 8,354,449.

Also disclosed herein is a process for preparing an L- or D-isomer of the compounds of the present invention are provided, comprising adding a base to L- or D-cystine diamide dihydrochloride to produce a first mixture, and subsequently heating the first mixture under vacuum; adding a methanolic solution to the heated first mixture; acidifying the mixture with alcoholic hydrogen chloride to obtain a first residue; dissolving the first residue in a first solution comprising methanol saturated with ammonia; adding a second solution to the dissolved first residue to produce a second mixture; precipitating and washing the second mixture; filtering and drying the second mixture to obtain a second residue; mixing the second residue with liquid ammonia and an ethanolic solution of ammonium chloride to produce a third mixture; and filtering and drying the third mixture, thereby preparing the L- or D-isomer compound.

In some embodiments, the process further comprises dissolving the L- or D-isomer compound in ether; adding to the dissolved L- or D-isomer compound an ethereal solution of lithium aluminum hydride, ethyl acetate, and water to produce a fourth mixture; and filtering and drying the fourth mixture, thereby preparing the L- or D-isomer compound.

Also disclosed herein is a process for preparing an L- or D-isomer of the compounds disclosed herein, comprising mixing S-benzyl-L- or D-cysteine methyl ester hydrochloride or O-benzyl-L- or D-serine methyl ester hydrochloride with a base to produce a first mixture; adding ether to the first mixture; filtering and concentrating the first mixture; repeating steps (c) and (d), to obtain a first residue; adding ethyl acetate and a first solution to the first residue to produce a second mixture; filtering and drying the second mixture to produce a second residue; mixing the second residue with liquid ammonia, sodium metal, and an ethanolic solution of ammonium chloride to produce a third mixture; and filtering and drying the third mixture, thereby preparing the L- or D-isomer compound.

### Cataracts

According to certain embodiments, NACA and derivatives thereof are used in the treatment of cataracts. In certain embodiments, the cataracts are caused by age, trauma, UV radiation exposure, genetics, skin diseases or medications. Cataracts include nuclear sclerosis, cortical cataract and posterior subcapsular cataract. Catracts that can be treating according to the methods disclosed herein include cataracts of various grades. Generally, grades show describe cataracts of increasing severity. In certain embodiments, the Lens Opacity Classification System III (LOCS III) is used to grade cataracts. This system grades cataracts as nuclear, anterior or posterior and uses a severity scale of 1-5, with 1 the least severe and 5 the most severe.

In certain embodiments, the administration of NACA or derivatives thereof leads to a reduction in the severity of cataracts. Thus, in certain embodiments, the LOCS III grade of the cataract would be reduced after administration of NACA or derivatives thereof. In certain embodiments, the cataracts are reduced in severity by one, two, three or four grades. In certain embodiments, administration of NACA or derivatives thereof leads to elimination of the cataract. In other embodiments, administration of NACA and derivatives thereof leads to stabilization of a cataract that is worsening in severity. Thus, a subject that has a cataract of a certain grade, that would have a more severe cataract without the administration of NACA or a derivative thereof retains a cataract of the same severity after administration of NACA or a derivative thereof.

According to certain embodiments, NACA or derivatives thereof can be administered systemically or on, in or near the eye to subjects suffering from cataracts. Systemic administration methods include intraperitoneal, intravenous or oral administration. Administration onto the eye includes administration through eye drops or ointments appropriate for administration onto the eye. Administration into the eye can be performed using intraocular injection. Administration near the eye can be performed using periocular injection. However, any intraocular administration method known in the art could be used.

Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg per dose, or in an amount equivalent to at least 50-350 mg per dose, or in an amount equivalent to at least 50-150 mg per dose, or in an amount equivalent to at least 25-250 mg per dose, or in an amount equivalent to at least 50 mg per dose. NAC amide or a derivative thereof can be administered to both human and non-human mammals. It therefore has application in both human and veterinary medicine.

In certain embodiments, treatment with NACA can last for 1, 2, 3, 4, 5, 10, 15, 20, 25, 30, 35, 40, 45 or 50 weeks. NACA can be administered chronically to prevent the onset of cataracts or to prevent worsening of cataracts. In certain embodiments, NACA is administered in eye drops. These eye drops can further include a pharmaceutically acceptable buffer.

### Spinal Cord Injury (not within the scope of the claims)

In certain embodiments, NACA and derivatives thereof can be used for the treatment of nervous tissue injury. In some embodiments, the nervous tissue injury is a spinal cord injury. According to some embodiments, injury is ameliorated through increasing the mitochondrial respiratory controlled ration (RCR) and/or the respiration rate of cells in the injured tissued. In some embodiments, the RCR and respiration rate can be increased in neuronal or non-neuronal cells from the spinal cord. Non-neuronal or non-synaptic cells include neuronal soma and glial cells. In certain embodiments, the RCR or respiration rates of neuronal cells are increased. In other embodiments, the RCR or respiration rates of non-neuronal cells are increased. In other embodiments, the RCR or respiration rates of neuronal and non-neuronal cells are increased.

Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 75-600 mg/kg per dose, or in an amount equivalent to at least 200-400 mg/kg per dose, or in an amount equivalent to at least 250-350 mg/kg per dose, or in an amount equivalent to about 300 mg/kg per dose, or in an amount equivalent to at least 50 mg per dose. NAC amide or a derivative thereof can be administered to both human and non-human mammals. It therefore has application in both human and veterinary medicine.

According to certain embodiments, NACA or derivatives thereof can be administered systemically or on, in or near the eye to subjects suffering from cataracts. Systemic administration methods include intraperitoneal, intravenous or oral administration. NACA and derivatives thereof can also be administered directly to the damaged nerve tissue. Thus, NACA or derivatives thereof can be injected into spinal cord or into the brain or administered directly to other nerve tissue. NACA or derivatives thereof may also be administered by any method known in the art.

In certain embodiments, treating the injury to nervous tissue can be used to improve paralysis or lack of sensation in a subject. In some embodiments, the nervous tissue damage can be the result of a stroke, aneurysm or trauma. In certain embodiments, the nervous tissue damage can lead to lack of function of the limbs, including leg paralysis. In these embodiments, administration of therapeutically effective amounts of NACA or derivatives thereof leads to the reduction of paralysis and increase in normal function of the limbs. Limbs can include arms and legs.

In some embodiments, cells in injured nerve tissue have impaired mitochondrial oxygen consumption rate in State III (ATP phosphorylation) and/or State V (complex I). Administration of effective amounts of NACA or derivatives thereof can increase the mitochondrial oxygen consumption rate in State III (ATP phosphorylation) and/or State V (complex I) after nerve tissue injury. In other embodiments, injured nerve tissue have impaired overall mitochondrial oxygen consumption rate. Administration of effective amounts of NACA or derivatives thereof can increase the overall mitochondrial oxygen consumption rate after nerve tissue injury. In other embodiments, nerve tissue injury leads to reduction of enzymatic activity in one or more of NADH dehydrogenase (Complex I); Cytochrome c Oxidase (Complex IV); and/or Pyruvate dehydrogenase complex (PDHC). Administration of effective amounts of NACA or derivatives thereof can increase any one or more of these activities after nerve tissue injury.

### HIV (not within the scope of the claims)

In certain embodiments, NACA and derivatives thereof are used for the treatment of human immunodeficiency virus (HIV) infection and symptoms associated with acquired immune deficiency syndrome (AIDS). In some embodiments, NACA and derivatives thereof are used to reduce HIV replication in infected cells. These cells can include peripheral blood mononuclear cells (PBMCs). PDMCs include lymphocytes, monocytes and macrophages. Lymphocytes include B-cells and T-cells. T-cells include CD4 and CD8 positive T-cells. NACA and derivatives thereof can be used to reduce HIV replication in any of these cell types. In certain embodiments, replication is reduced, greater than 10, 15, 20, 25, 30, 35, 40, 45, 50, 55, 60, 65, 70, 75, 80, 85, 90, 95, 96, 97, 98, 99 or 100%.

In certain embodiments, NACA and derivatives thereof can be used to reduce reverse transcriptase (RT) activity in HIV infected cells. These cells can include peripheral blood mononuclear cells (PBMCs). PDMCs include lymphocytes, monocytes and macrophages. Lymphocytes include B-cells and T-cells. T-cells include CD4 and CD8 positive T-cells. NACA and derivatives thereof can be used to reduce RT activity in any of these cell types. In certain embodiments, RT activity is reduced, greater than 10, 15, 20, 25, 30, 35, 40, 45, 50, 55, 60, 65, 70, 75, 80, 85, 90, 95, 96, 97, 98, 99 or 100%.

According to certain embodiments, NACA or derivatives thereof can be administered systemically. Systemic administration methods include intraperitoneal, intravenous or oral administration. Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg per dose, or in an amount equivalent to at least 50-350 mg per dose, or in an amount equivalent to at least 50-150 mg per dose, or in an amount equivalent to at least 25-250 mg per dose, or in an amount equivalent to at least 50 mg per dose.

In some embodiments, administration of NACA or derivatives thereof to reduce symptoms associated with HIV infection in a subject in need thereof. Administration of therapeutically effective amount of NACA or a derivative thereof can improve symptoms associated with AIDS. These symptoms include various immune dysfunctions that can present as fever, fatigue, swollen lymph nodes, diarrhea, weight loss, cough, shortness of breath, night sweats, chills and skin pathology. In other embodiments, NACA or derivatives thereof can be co-administered with any known HIV or AIDS therapeutic. These therapeutics include fusion inhibitors, CCR5 receptor antagonists, nucleoside reverse transcriptase inhibitors, non- nucleoside reverse transcriptase inhibitors, protease inhibitors, integrase inhibitors and maturation inhibitors.

### Oncolytic Viral Therapy (not within the scope of the claims)

In certain embodiments, NACA or derivatives thereof are used to increase the effectiveness of oncolytic viruses. Oncolytic viruses can be used to reduce the viability of various tumor cells. These tumor cells can be from cancers including glioma, breast cancer, lung cancer, brain cancer, melanoma, prostate cancer, ovarian cancer, pancreatic cancer, liver cancer, colon cancer, cervical cancer, bladder cancer, spleen cancer, head and neck cancer, or bone cancer. In certain embodiments, NACA or derivatives thereof can be administered with oncolytic virsues for the treatment of glioma. In some embodiments, the glioma is glioblastoma multiforme (GBM).

In certain embodiments, oncolytic viruses are administered in carrier cells. The carrier cells can be transformed with the oncolytic viruses and then administered to a subject in need thereof. The oncolytic viruses can then be contacted with cancer cells to reduce their viability. In some embodiments, NACA or derivatives thereof can be used to increase the viability of the carrier cells. This allows a given dose of carrier cells transformed with oncolytic viruses the ability to delivery more virus to tumor cells thus decreasing the viability of the tumor cells. In certain embodiments, administration of NACA and derivatives thereof with carrier cells reduced reactive oxygen species in the carrier cells.

According to certain embodiments, NACA or derivatives thereof along with carrier cells including oncolytic viruses can be administered systemically or on, in or near the tumor to be treated in a subject. Systemic administration methods include intraperitoneal or intravenous administration. Administration into a tumor can be performed by injecting NACA or derivatives thereof along with carrier cells including oncolytic viruses at the site of the tumor. With glioma this could mean injection into the brain of the subject. In certain embodiments, the carrier cells including oncolytic viruses are administered at the site of the tumor while the NACA or derivatives thereof are administered systemically. Systemic administration methods include intraperitoneal, intravenous or oral administration.

Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg/kg per dose, or in an amount equivalent to at least 50-350 mg/kg per dose, or in an amount equivalent to at least 100-400 mg/kg per dose, or in an amount equivalent to at least 200-300 mg/kg per dose, or in an amount equivalent to about 250 mg/kg per dose. NACA or a derivative thereof can be administered to both human and non-human mammals. It therefore has application in both human and veterinary medicine. The carrier cells including oncolytic viruses can be administered at between 1x10⁵ cells to 1x10⁸ cells. In certain embodiments, between 1x10⁵ and 10x10⁵ cells can be administered.

According to certain embodiments, administration of NACA or derivatives thereof with carrier cells including oncolytic viruses increases the viability of the carrier cells by 10, 15, 20, 25, 30, 35, 40, 45, 50, 55, 60, 65, 70, 75, 80, 85, 90, 95, 100, 150, 200, 300, 400, 500, 600, 700, 800, 900 or 1000%. In certain embodiments, administration of NACA or derivatives thereof with carrier cells including oncolytic viruses increases the viability of the carrier cells by between 20 and 50% or between 50 and 500%.

According to other embodiments, administration of NACA or derivatives thereof with carrier cells including oncolytic viruses decreaes the viability of the targeted cancer cell by at least 10, 15, 20, 25, 30, 35, 40, 45, 50, 55, 60, 65, 70, 75, 80, 85, 90, 95, 100%. In certain embodiments, administration of NACA or derivatives thereof with carrier cells including oncolytic viruses decreaes the viability of the targeted cancer cell by between 20 and 75% or between 25 and 50%.

In certain embodiments, the oncolytic virus is CRAd-S-pk7. In other embodiments, the carrier cell is a neural stem cell. In other embodiments, the neural stem cell is an immortalized stem cell.

### Stem Cells (not within the scope of the claims)

In certain embodiments, NACA or derivatives thereof are used to increase the viability of stem cells. Stem cells that can be used according to these methods include pluripotent, totipotent, multipotent, oligopotent or unipotent stem cells. Stem cells can also include embryonic, fetal or adult stem cells. In other embodiments, stem cells can include mesenchymal stem cells, skin stem cells, muscle stem cells, neuronal stem cells and bone stem cells. In certain embodiments, stem cells used according to the methods described herein are neuronal stem cells.

According to certain embodiments, NACA can be administered on stem cells *in vitro* or *in vivo.* When administered *in vivo* NACA can be administered systemically or at the site at which the stem cells are present. When administered systemically, NACA can be adminstered intravenously, intraperitoneally or orally. In certain embodiments, NACA is first administered to stem cells *in vitro* and the stem cells are then administered to a subject. In other embodiments, stem cells are administered to a subject with NACA. In these embodiments, the NACA and stem cells can be injected at the same time or administered via different routes. For example, stem cells could be injected at a particular site in a subject and NACA could be administered systemically.

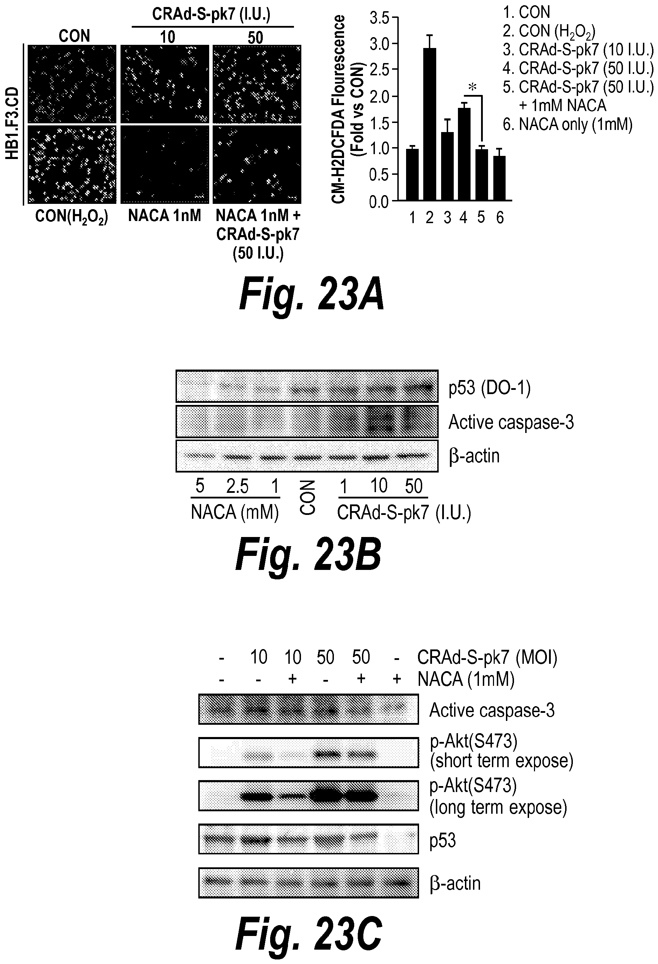

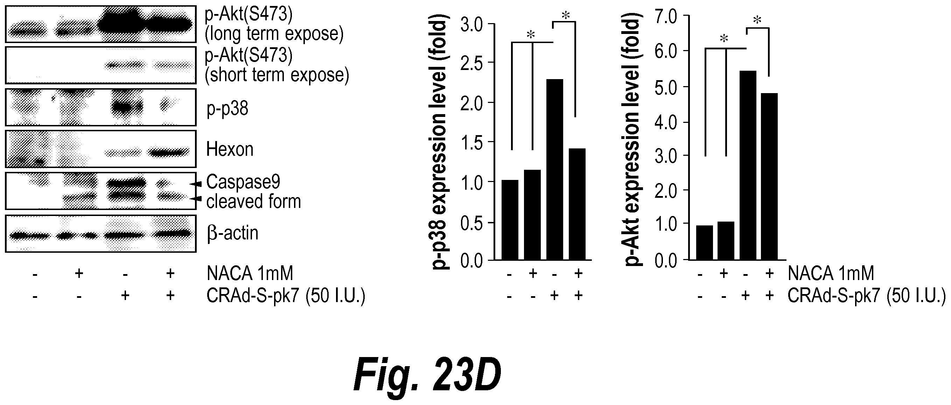

As shown in Figure 23, reactive oxygen species (ROS) accumulated in a dose-dependent manner. Treatment with NACA, however, effectively decreased endogenous ROS levels that were activated by cellular loading with oncolytic viruses. An interesting finding was that non-infected NSCs that were used as a control inherently expressed high levels of intracellular ROS. Such overexpression was decreased by NACA treatment.

Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg/kg per dose, or in an amount equivalent to at least 50-350 mg/kg per dose, or in an amount equivalent to at least 100-400 mg/kg per dose, or in an amount equivalent to at least 200-300 mg/kg per dose, or in an amount equivalent to about 250 mg/kg per dose. NACA or a derivative thereof can be administered to both human and non-human mammals. It therefore has application in both human and veterinary medicine. The stem cells can be administered at between 1x10⁵ cells to 1x10⁸ cells. In certain embodiments, between 1x10⁵ and 10x10⁵ cells can be administered.

According to certain embodiments, administration of NACA or derivatives thereof with increases the viability of stem cells by 10, 15, 20, 25, 30, 35, 40, 45, 50, 55, 60, 65, 70, 75, 80, 85, 90, 95, 100, 150, 200, 300, 400, 500, 600, 700, 800, 900 or 1000%. In certain embodiments, administration of NACA or derivatives thereof increases the viability of stem cells by between 20 and 50% or between 50 and 500%.

### Asphyxia (not within the scope of the claims)

In certain embodiments, NACA or derivatives thereof are used for the treatment of asphyxia. In some embodiments, asphyxia is treated when a subject receives increased oxygenation of the brain due to administration of NACA or a derivative thereof than the subject would have otherwise received. In other embodiments, administration of NACA or a derivative thereof increases oxygen to the brain in hypoxemic conditions. Hypoxemic conditions are those wherein the ambient oxygen level available to breathe is less than the concentration of oxygen at sea level. In certain embodiments, NACA or derivatives thereof can be administered in situations where a subject is likely to encounter low oxygen conditions, such as at high altitude or in an air plane to increase oxygenation of the brain.

According to certain embodiments, NACA or derivatives thereof can be administered systemically. Systemic administration methods include intraperitoneal, intravenous or oral administration. Doses, amounts or quantities of NACA, or derivatives thereof, are determined on an individual basis. As is appreciated by the skilled practitioner in the art, dosing is dependent on the severity and responsiveness of the cataract to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg per dose, or in an amount equivalent to at least 50-350 mg per dose, or in an amount equivalent to at least 50-150 mg per dose, or in an amount equivalent to at least 25-250 mg per dose, or in an amount equivalent to at least 50 mg per dose. NACA or a derivative thereof can be administered to both human and non-human mammals. It therefore has application in both human and veterinary medicine.

### Pharmaceutical Compositions

As used herein the term "pharmaceutical composition" refers to a preparation of one or more of the components described herein, or physiologically acceptable salts or prodrugs thereof, with other chemical components such as physiologically suitable carriers and excipients. The purpose of a pharmaceutical composition is to facilitate administration of a compound to an organism. The term "prodrug" refers a precursor compound that can hydrolyze, oxidize, or otherwise react under biological conditions (in vitro or in vivo) to provide the active compound. Examples of prodrugs include, but are not limited to, metabolites of NSAIDs that include biohydrolyzable moieties such as biohydrolyzable ainides, biohydrolyzable esters, biohydrolyzable carbamates, biohydrolyzable carbonates, biohydrolyzable ureides, and biohydrolyzable phosphate analogues.

The term "excipient" refers to an inert or inactive substance added to a pharmaceutical composition to further facilitate administration of a compound. Non-limiting examples of excipients include calcium carbonate, calcium phosphate, various sugars and types of starch, cellulose derivatives, gelatin, vegetable oils and polyethylene glycols.

The pharmaceutical compositions of the present invention comprise NAC Amide or derviate thereof and may also include one or more additive drug (e.g., additional active ingredients), such as, but not limited to, NSAIDs, antibiotics, conventional anti-cancer and/or anti-inflammatory agents that may be suitable for combination therapy.

The pharmaceutical compositions of the present invention may be manufactured by processes well known in the art, e.g., by means of conventional mixing, dissolving, granulating, grinding, pulverizing, dragee-making, levigating, emulsifying, encapsulating, entrapping or by lyophilizing processes.

The compositions for use in accordance with the present invention thus may be formulated in conventional manner using one or more pharmaceutically acceptable carriers comprising excipients and auxiliaries, which facilitate processing of the active compounds into preparations which can be used pharmaceutically. Proper formulation is dependent upon the route of administration chosen.

The term "administration" or any lingual variation thereof as used herein is meant any way of administration. The one or more of NAC Amide or derivative thereof and at least one additional drug may be administered in one therapeutic dosage form or in two separate therapeutic dosages such as in separate capsules, tablets or injections. In the case of the two separate therapeutic dosages, the administration may be such that the periods between the administrations vary or are determined by the practitioner. It is however preferred that the second drug is administered within the therapeutic response time of the first drug. The one or more of NAC Amide or derivative thereof and at least one additional drug which may be administered either at the same time, or separately, or sequentially, according to the invention, do not represent a mere aggregate of known agents, but a new combination with the valuable property that the effectiveness of the treatment is achieved at a much lower dosage of said at least one additional drug.

The pharmaceutical compositions of the present invention may be administered by any convenient route, for example, by infusion or bolus injection, by absorption through epithelial or mucocutaneous linings (e.g., oral mucosa, rectal and intestinal mucosa, etc.) and may be administered together with any other therapeutic agent. Administration can be systemic or local.

Various delivery systems are known, e.g., encapsulation in liposomes, microparticles, microcapsules or capsules, that may be used to administer the compositions of the invention. Methods of administration include but are not limited to intradermal, intramuscular, intraperitoneal, intravenous, subcutaneous, intranasal, epidural, oral, sublingual, intranasal, intracerebral, intravaginal, transdermal, rectally, by inhalation, or topically to the cars, nose, eyes, or skin. The preferred mode of administration is left to the discretion of the practitioner, and will depend in part upon the site of the medical condition (such as the site of cancer) and the severity of thereof.

For example, for injection the composition of the invention may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants for example DMSO, or polyethylene glycol are generally known in the art.

For oral administration, the composition can be formulated readily by combining the active components with any pharmaceutically acceptable carriers known in the art. Such "carriers" may facilitate the manufacture of such as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum, methyl cellulose, hydroxypropylmethyl-cellulose, sodium carbomethylcellulose, and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active components may be dissolved or suspended in suitable liquids, such as fatty oils, liquid paraffin, or liquid polyethylene glycols.

Dyestuffs or pigments may be added to the tablets or dragee coatings for identification or to characterize different combinations of active NSAID doses. In addition, stabilizers may be added.

Pharmaceutical compositions for parenteral administration include aqueous solutions of the active preparation in a water-soluble form. Additionally, suspensions of the active preparation may be prepared as oily injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl, cellulose, sorbitol or dextran. Optionally, the suspension may also contain suitable stabilizers or agents, which increase the solubility of the compounds, to allow for the preparation of highly concentrated solutions.

Alternatively, the composition may be in a powder form for constitution before use with a suitable vehicle, e.g., sterile, pyrogen-free water. The exact formulation, route of administration and dosage may be chosen by the physician familiar with the patient's condition. (See for example Fingl, et al., 1975, in "The Pharmacological Basis of Therapeutics", Chapter I, p. 1). Depending on the severity and responsiveness of the condition treated, dosing can also be a single administration of a slow release composition, with course of treatment lasting from several days to several weeks or until cure is effected or diminution of the disease state is achieved.

### EXAMPLES

### Example 1: NACA effects on cataracts.

Experiment 1: L-buthionine sulfoximine (BSO) was given to rats to induce cataracts for 3 days via i.p injection (injections performed on day 3). NACA given via i.p. injections gave lesser grade of cataracts in all rats as compared to the untreated group. 80% of lens in the NACA treated group showed no signs of cataract formation.

Experiment 2: BSO was given to rats to induce cataracts for 3 days via i.p. injection (injections performed on day 10). NACA given via eye drop to group of rats after forming grade I cataracts. NACA given via eye drop to group of rats after forming grade II cataracts. All rats with grade I cataracts prior to NACA eye drop treatment showed no signs of cataracts after 3 week treatment. All rats with grade II cataracts prior to NACA eye drop treatment showed a reduction in grade of cataract after 3 week treatment (75% of which showed no sign of cataracts). BSO-only group also showed a reduction in the grade of cataract (All rats had a grade between 0 and II -- no grade III cataracts were present and 1-2 lens had no signs of cataract formation).

Dose/Methods: For the NACA application with eye drops, we used a 1% solution in Phosphate buffer solution (100mM) @ pH 7.0. The eye drops were applied twice daily 12 hours between each scheduled dose. Two drops were applied in each eye consisting of 10 µL each. The results shown were treatment given for 3 weeks after the corresponding grade cataract was formed.

To generate the cataracts, an i.p. injection of (4mM/kg body weight) Buthathione Sulfoxamine (BSO) was administered twice, the first on 9 days of age and the second on 10 days of age.

There were 8 rats which had grade II cataracts and 12 rats which had grade I cataracts upon 15 days of age.

### Reference Example 2: NACA in spinal cord injury model.

**Summary:** Female SD rats were contused at upper lumbar (L1-L2) spinal cord level at 250 kDyn. Rats were treated with N-acetyl cysteine amide (DP1, DP1 was supplied from David Pharmaceuticals), a glutathione precursor or vehicle (vehicle) 15 min post-injury (150 mg/kg in saline) followed by a booster at 6 hr post-injury (n=4/group). Spinal cords were dissected out 24 hr after injury and 1.5 cm tissue segment centered on epicenter were taken for mitochondrial isolation. Isolated mitochondria were subjected for assessment of mitochondrial respiration rates.

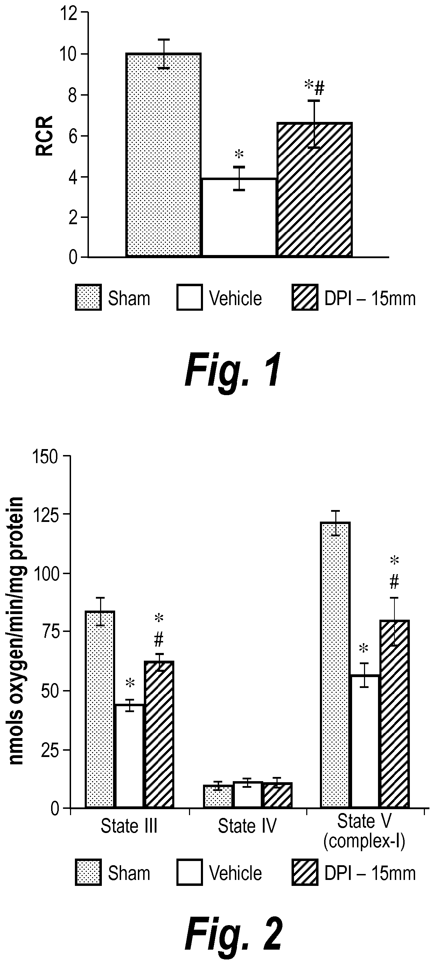

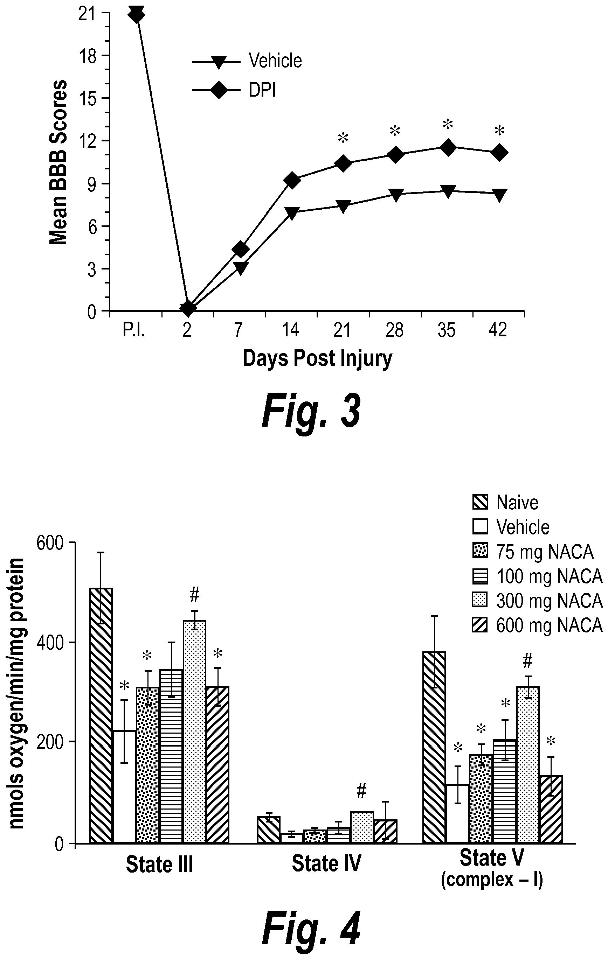

Results shows that mitochondrial respiratory controlled ratio (RCR) and respiration rates significantly decreased following injury compared to shams (Figures 1 and 2). Treatment with DP1 significantly improved RCR and respiration rates compared to vehicle treatment (Figures 1 and 2). In a long-term behavior study, we similarly administered DP1 or saline at 15 min post injury followed by 6 hr booster. We also inserted subcutaneous osmotic pumps containing DP1 or saline to continually deliver for 7-days post-injury (n=5/group). As illustrated in Figure 3, after 6 weeks of testing, treatment with DP1 rendered significant increases in the BBB scores (∼11-walking) compared to vehicle treatment (∼8-dragging limbs). Overall, DP1 treatment promotes neuroprotection by targeting mitochondrial dysfunction that is directly correlated with the improved hind-limb function over weeks post-injury.

### Reference Example 3: NACA As a Mitochondrial-Targeted Therapeutics for Treatment of Spinal Cord Injury.

The neuroprotective efficacy of a glutathione precursor, N-acetylcysteine amide (NACA) was evaluated following contusion spinal cord injury (SCI). Adult female SD rats were contused (250 kdyn @ L1/L2) using IH impactor. Vehicle (saline) or one of the 4 dosages of NACA (75, 150, 300 and 600 mg/kg) were administered (i.p.) 15 min post-injury, followed by one booster at 6 hrs post-injury. At 24 hr post-injury, synaptic (neuronal), non-synaptic (neuronal soma and glia) and total (synaptic and non-synaptic) mitochondria from naive and injured spinal cords were isolated and assessed for mitochondrial respiration and activities of NADH dehydrogenase, cytochrome oxidase and pyruvate dehydrogenase. Compared to the naive group, SCI resulted in significantly compromised mitochondrial bioenergetics. NACA treatment improved mitochondrial bioenergetics with maximum restoration (p<0.05) at 300 mg/kg. Next, hindlimb functional recovery and tissue sparing was studied using a NACA dose of 300 mg/kg. Vehicle or NACA was administered 15 min post-injury. Osmotic pumps (s.c.) were inserted (delivery rate 300 mg/day) for 7 days post-injury. NACA treatment improved hindlimb locomotor function beginning 7 days-post SCI. After 7 weeks, NACA-treated rats were consistently stepping with weight support and displayed frequent coordination compared to vehicle-treated rats that only demonstrated frequent dorsal and occasional planter hindlimb stepping. Ongoing experiments include quantitative analysis of glutathione and oxidative markers of mitochondria isolated from acute experimental groups, and histological assessments following prolonged NACA treatment. Collectively, these results showed that acute NACA treatment after SCI significantly maintains mitochondrial bioenergetics in all three mitochondrial populations, and prolonged continuous treatment with NACA significantly improves the recovery of hindlimb locomotor function. This study was supported by KSCHIRT #8-13 (AGR), NIH/NINDS R01NS069633 (AGR & PGS), NIH/NINDS P30 NS051220 and a generous donation from the Michael and Helen Schaffer Foundation, Boston, MA.

In pilot experiments, using naive spinal cord mitochondria, methods were standardized for measurement of mitochondrial oxygen consumption employing the Seahorse Biosciences XF24 Flux Analyzer, a multiple microplate based system. This revealed that only ∼5µg mitochondrial protein is needed to measure oxygen consumption compared to the Clark-type oxygen electrode which required ∼80µg of mitochondrial protein; a 16-fold yield increase. In addition to increased sensitivity, multiple samples can be analyzed at the same time.

Therefore, using the 24 well Seahorse XF24 Flux Analyzer, mitochondrial respiration was assessed in synaptic mitochondria isolated 24 hrs after spinal cord injury (SCI). Injured rats received i.p. injection of either Vehicle (saline) or one of the four different dosages of NACA (75, 150, 300 and 600 mg/kg body weight) at 15 min post-SCI, followed by a booster (same respective dosage of either NACA or vehicle) at 6 hr after first administration. Compared to naive mitochondria, significantly (p<0.05) impaired mitochondrial oxygen consumption rate (OCR) [State III (ATP phosphorylation) & State V (complex I)] was observed after SCI with Vehicle treatment (Figure 4). In comparison, NACA treatment at all the dosages improved mitochondrial OCR and showed a bell shape curve, with maximum restoration (p<0.05) of OCR with the 300 mg/kg dosage. However, mitochondrial OCR after 75, 150 and 600 mg/kg body weight NACA treatment remained significantly lower than naive.

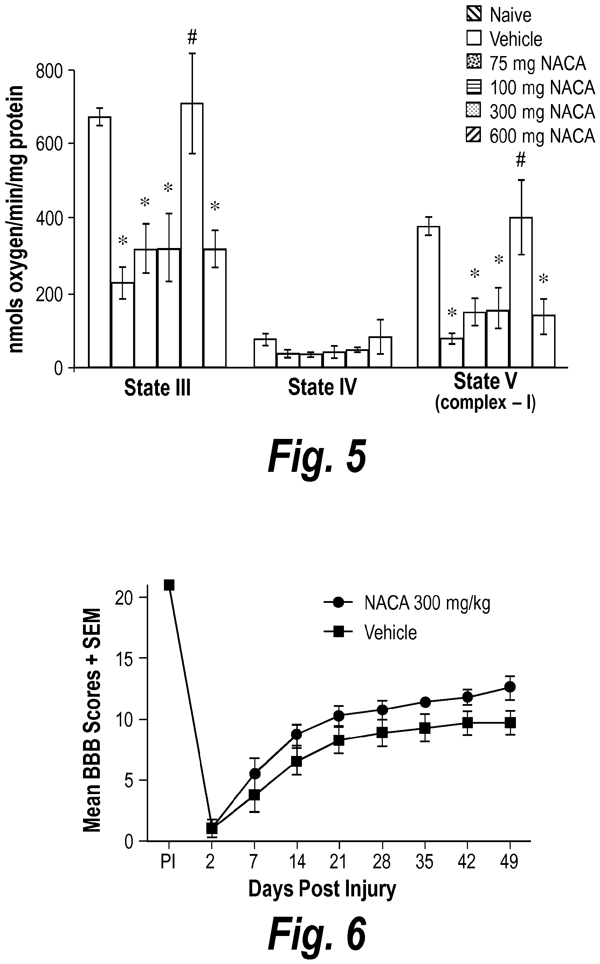

Similar to synaptic mitochondria, we found that SCI resulted in significantly (p<0.05) impaired mitochondrial OCR (State III-ATP phosphorylation) in non-synaptic mitochondria. Compared to vehicle, NACA treatment at 300 mg/kg body weight significantly (p<0.05) restored mitochondrial OCR. While 75, 150 and 600 mg/kg body weight NACA treatment improved OCR, they were not significantly different from injured Vehicle treatment values (Figure 5). Therefore, based on these collective results, 300 mg/kg body weight NACA will be used in Aim 2 for combinatorial treatment with ALC.

Based on the respiration data above, in the next set of completed experiments two dosages of NACA (75 or 300 mg/kg body weight) were selected to assess activities of mitochondrial enzyme complexes 1) NADH dehydrogenase (Complex I), 2) Cytochrome c Oxidase (Complex IV) and 3) Pyruvate dehydrogenase complex (PDHC). Results showed that compared to naive, SCI resulted in significant (p<0.05) reduction in activities of all three enzyme complexes in both synaptic and non-synaptic mitochondria. However, treatment with NACA significantly improved their activities in a dose-dependent manner. Notably, the protective effects of NACA on complex I activity were more pronounced in non-synaptic mitochondria than synaptic mitochondria.

We assessed long-term hind limb functional recovery using the most effective NACA dose (300 mg/kg body weight) that we determined in mitochondrial respiration experiments. Vehicle or NACA (300 mg/kg body weight) were administered 15 min post-injury. Osmotic pumps (s.c.) were also inserted (delivery rate 300 mg/day) for 7 days post-injury. Treatment with NACA improved hindlimb locomotor function beginning 7 days-post SCI (see Figure 6). Critically, after 7 weeks the NACA-treated injured rats were able to consistently step with weight support and frequent coordination compared to vehicle-treated rats that only demonstrated frequent dorsal and occasional planter stepping of hind limb. We have also performed 2D and 3D kinematic assessments at 7 weeks post-injury in these cohorts of injured animals; analysis is ongoing.

In preliminarily studies long-term hindlimb functional recovery was also assessed using two different dosages of NACA (150 and 300 mg/kg body weight). Vehicle or NACA were injected (i.p.) 15 min post-injury before osmotic pumps were inserted (s.c.) to deliver at 150 or 300 mg/kg/day for 7 days post-injury. After 6 weeks, the injured rats treated with either NACA dosage showed significantly improved hindlimb locomotor recovery compared to vehicle treatment.

Notably, the NACA-treated injured rats were able to consistently step with weight support and showed occasional forelimb-hindlimb coordination compared to vehicle-treated rats that demonstrated frequent dorsal and only occasional planter stepping of hindlimbs. Furthermore, terminal quantitative 2D kinematic gait analysis at 6 weeks post-injury confirmed improved hindlimb stepping patterns following NACA treatment.

MRI analysis was carried out on the same cohorts of injured animals. Preliminary quantitative assessments indicated reduced lesion volume with NACA treatment compared to vehicle.

Confirmatory histological assessments of the same injured spinal cord tissues showed significantly reduced lesion volume and increased tissue sparing at injury epicenter with NACA treatment; in addition to increased spared white matter volume.

We also implemented 2D and 3D kinematic analysis of hind limb movements, including learning of acquisition software, precise calibration of complex photographic equipment interfaced with computers, constructing the behavioral apparatus for testing, creating enormous files for each animal required for data acquisition, and algorithmic quantification of normal walking patterns in uninjured rats followed by refining methods to quantify altered regularity indices of coordinated walking after SCI.

### C. Significance

Collectively, we have shown that NACA treatment acutely following contusion SCI at the L1/L2 level significantly maintains mitochondrial bioenergetics in both non-synaptic and synaptic mitochondria. Importantly, among all the dosages of NACA assessed, 300 mg/kg bodyweight is most effective in preserving mitochondrial function 24 hrs following SCI. Moreover, prolonged continuous treatment with NACA (300 mg/kg for 7 day post-injury) significantly improves the recovery of hindlimb locomotor function. We have also developed a method for measurements of mitochondrial function using Seahorse XF24 Flux Analyzer that requires very small protein samples than previously required using the Clark-type oxygen electrode. We are now prepared to move forward with the behavioral studies employing 2D and 3D kinematic analysis of hind limb movements.

### Reference Example 4: Treatment of HIV with NACA.

In some embodiments, the present invention relates to a method for inhibiting HIV replication. Data is attached reporting the results of an HIV study in lymphocytes with 100% block of replication at 20 mM concentration.

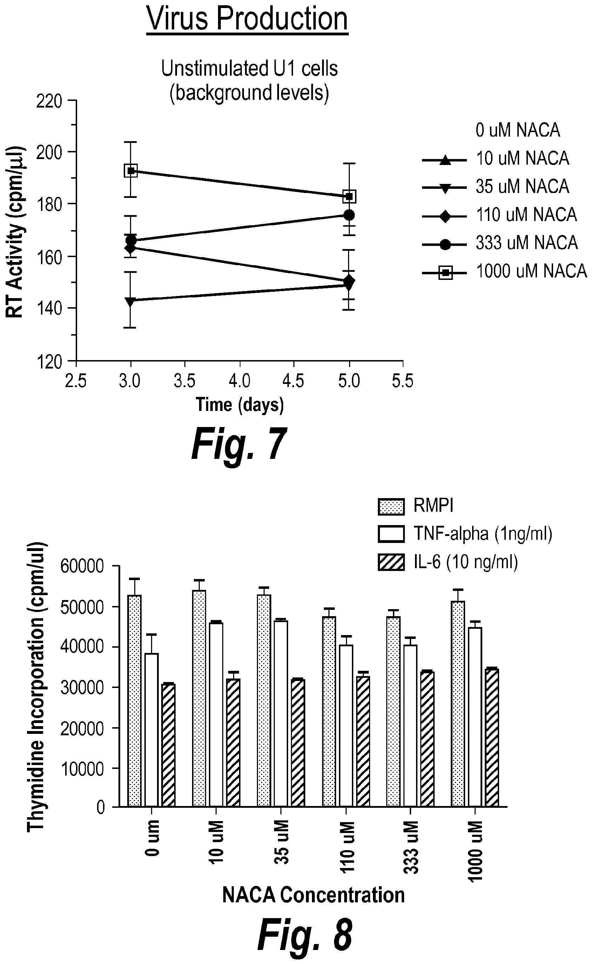

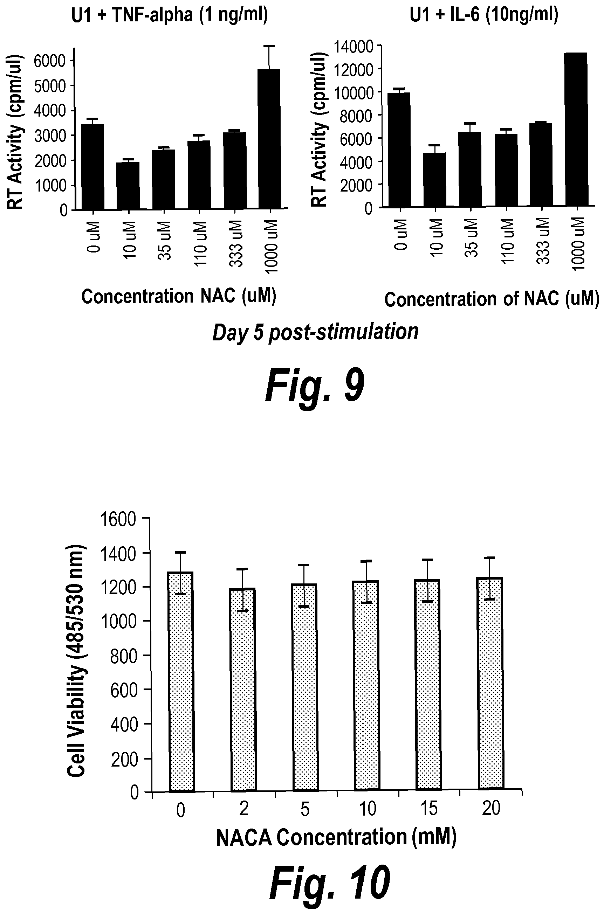

Chronically HIV-infected U1 cells (monocytes) were stimulated for 6 hours in the presence of NACA. Cytokines (IL-6, TNF-a) or PMA were added and maintained in the cell cultures for 6 days, collecting supernatants daily. Cell viability and proliferation were checked by optical microscopy, and [3H]-thymidine incorporation, and calcein AM assay. Reverse transcriptase-polymerase chain reaction (RT-PCR) and reverse transcriptase (RT) activity assay as readout of virion production. As shown in Figure 7, addition of NACA did not alter relative amounts of reverse transcriptase activity between days 3 and 5. Figure 8 shows that addition of NACA did not have a large effect on U1 cell viability or proliferation as measured by [3H]-thymidine incorporation. 10-333 µM NACA is effective in reducing HIV replication in activated U1 cells. Figure 9 shows significant reduction to reverse transcriptase activity in U1 cells activared with 1 ng/mL TNF-α or 10 ng/mL IL-6.

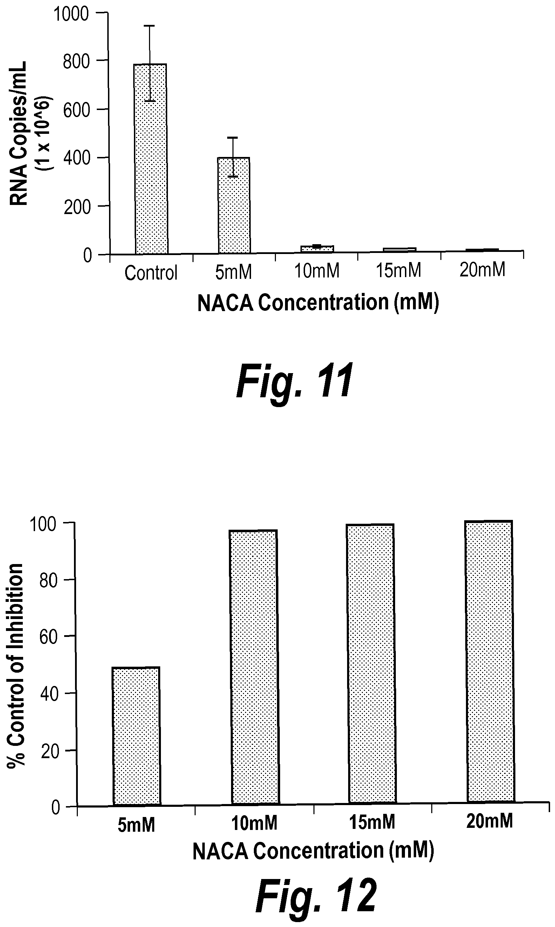

Peripheral blood mononuclear cells (PBMC) were acutely infected with HIV-1 and stimulated with NACA. Cell viability was measured in HIV infected PBMCs with varying concentrations of NACA. Figure 10 shows that administration of NACA did not affect cell viability. However, NACA had an inhibitor effect on HIV replication in HIV infected PBMCs as shown in Figure 11. At concentrations of 10 mM and above, HIV replication was almost 100% inhibited as shown in Figure 12.

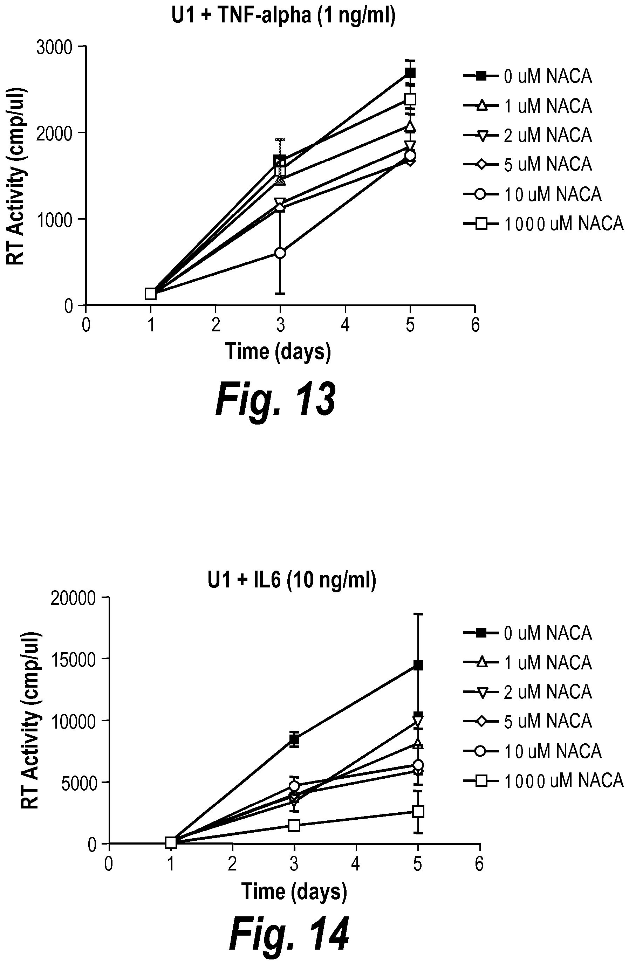

RT activity was also measured in activated U1 cells. U1 cells were activated with 1 ng/mL TNF-α, 10 ng/mL IL-6 or 10 nM phorbol 12-myristate 13-acetate (PMA). As shown in Figures 13-15, 1-10 µM NACA is effective in reducing HIV replication in activated U1 cells.

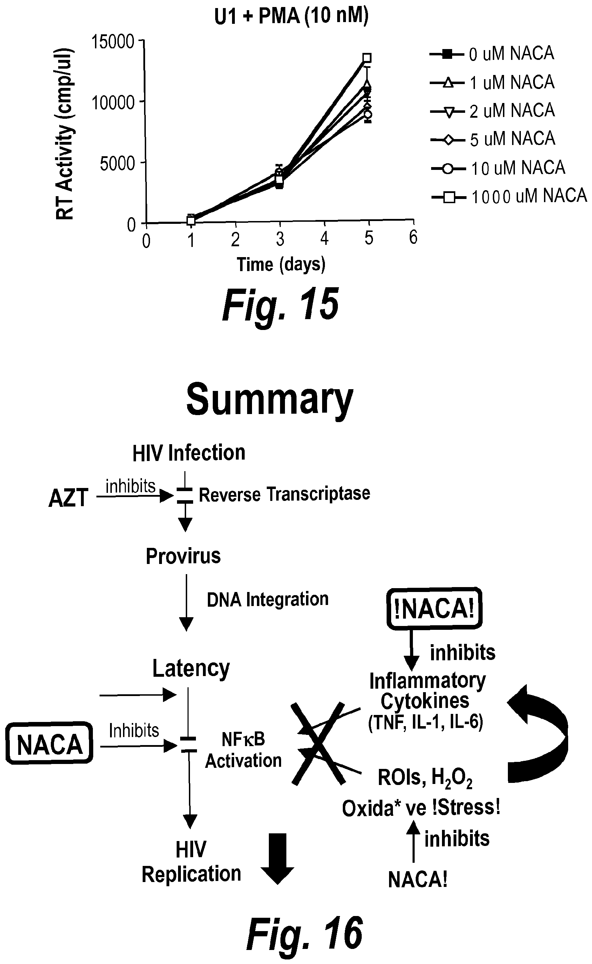

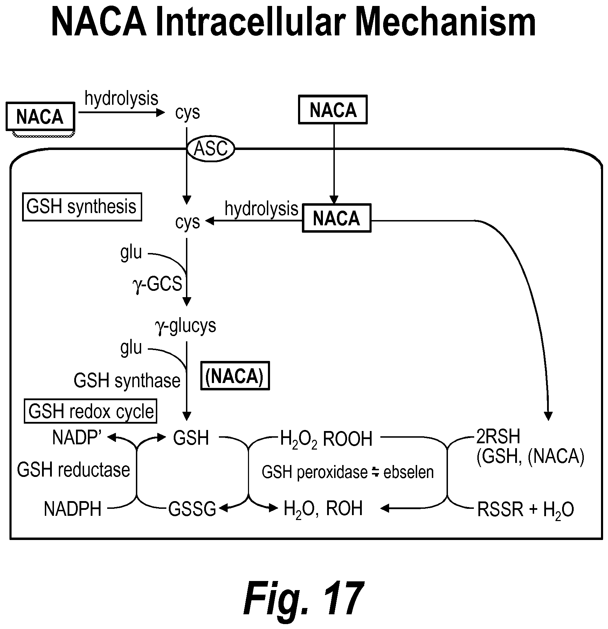

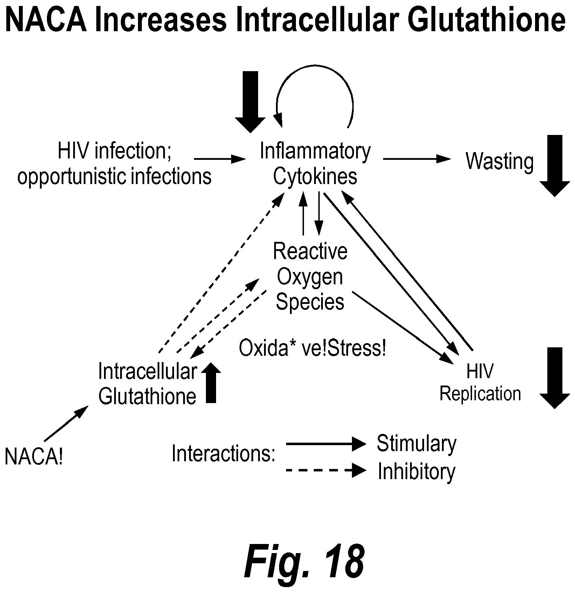

Applicants do not wish to be limited by theory, however it is possible that NACA inhibits HIV replication by preventing NFKB activation both by reducing reactive oxygen species and the effect of inflammatory cytokines as shown in Figure 16. NACA may also act by increasing intracellular glutathione as shown in Figures 17 and 18.

### Reference Example 5. N-acetylcysteine amide (NACA) Augments the Therapeutic Effect of Neural Stem Cell-Based Anti-Glioma Oncolytic Virotherapy

The experiments below show the role neural stem cells (NSCs) as delivery vehicles of CRAd-S-pk7, a gliomatropic oncolytic adenovirus (OV) and the role of *N*-acetylcysteine amide (NACA), in preventing OV-mediated toxicity towards NSC carriers in an orthotropic glioma xenograft mouse model. The results shown below demonstrate that the combination of NACA and CRAd-S-pk7 not only increases the viability of these cell carriers, but also improves the production of viral progeny in HB1.F3.CD NSCs. Furthermore, NACA treatment was able to reduce the production of endogenous ROS triggered by CRAd-S-pk7, thereby preventing ROS-induced apoptosis of NSCs. In an intracranial xenograft mouse model, the combination treatment of NACA and NSCs loaded with CRAd-S-pk7 showed enhanced mice survival and increased intratumoral apoptosis as compared to CRAd-S-pk7 loaded NSCs alone. The combined therapy enhanced CRAd-S-pk7 production and distribution in malignant tissues. Finally, these results suggest that NACA can increase the therapeutic efficacy of oncolytic viruses loaded into NSCs by preventing apoptosis and increasing viral production in the cell carrier. These data demonstrate that the combination of NACA and NSCs loaded with CRAd-S-pk7 may be a desirable strategy to improve the therapeutic efficacy of anti-glioma oncolytic virotherapy.

Glioblastoma multiforme (GBM) is the most common primary brain tumor in adults. Although several treatment options such as surgery, irradiation and chemotherapy have been attempted, the prognosis for GBM patients is still dismal due to the lack of therapeutic effectiveness. Even with aggressive and continued treatment, median survival of patients with GBM is only 12-15 months. The reason for such a poor prognosis is related to GBM's propensity for infiltration, invasion, and integration into normal brain tissues together with its inherent resistance to multimodal treatments. Another important consideration for poor outcome in patients with GBM is the presence of an ultra-selective blood-brain barrier (BBB), which hinders drug delivery to brain tumors. This is considered to be one of main problems of systemic chemotherapy against GBM. Therefore, novel feasible therapeutic strategies that are able to specifically target gliomas and overcome the above-described limitations need to be developed in order to prevent GBM recurrence and enhance the prognosis of affected patients.

Oncolytic virus (OV) therapy with replication-competent viruses is an attractive tool for the treatment of malignant cancers. In order to reach a feasible clinical application, OVs need to be safe and nontoxic to normal cells while capable of selectively destroying cancer cells. The CRAd-S-pk7 oncolytic virus is a conditionally replicative adenovirus (CRAd) vector that employs the survivin (S) promoter and a fiber modification containing polylysine (pk7)) to selectively transduce and replicate in neoplastic cells. It has previously been shown that CRAd-S-pk7 enhances cell cycle arrest, apoptosis, and oncolytic effect in glioma cells. It has also been demonstrated that CRAd-S-pk7 oncolytic virotherapy either alone or in combination with temozolomide, a well-known chemotherapeutic agent, increases the survival of mice bearing intracranial glioma xenografts. Although successful preclinical results have been obtained with oncolytic adenoviruses in anti-glioma therapy, this therapeutic strategy is still limited due to the rapid OV clearance by the host immune system and inefficient viral delivery to the tumor sites.

To overcome this limitation, an FDA approved immortalized NSC line has been used for human clinical trials, HB1.F3.CD, as a delivery vehicle of OVs. This carrier is able to home to tumor areas and evade host immune response elicited by virus infection. We have shown that CRAd-S-pk7 loaded HB1.F3.CD NSCs were able to suppress anti-adenoviral immune response and enhance anti-glioma therapeutic efficacy compared to CRAd-S-pk7 alone. Additional *in vivo* studies have demonstrated that CRAd-S-pk7 loaded HB1.F3.CD cells supported virus replication and maintained their tumor tropic properties for more than a week. In order to enhance the therapeutic efficacy of oncolytic vectors, various preclinical studies have investigated the use of combined anti-cancer drugs with OVs in glioma-bearing animal models. Recently, it was reported that the combination of oncolytic Herpes Simplex Virus-1 (oHSV-1) and copper inhibitor (ATN-224) significantly decreased glioma growth and prolonged animal survival. An additional report has shown that oHSV combined with low-dose-Etoposide was able to increase the survival of cancer stem cell-enriched glioma-bearing mice. Taken together, the above results suggest that the combination of oncolytic viruses and chemotherapeutic drugs can provide a potential strategy for anti-glioma therapy.

It has been previously shown that oxidative stress (OS) plays a critical role in many biological pathways such as programmed cell death, age-related diseases, tumorigenesis as well as autophagy. In response to viral infection, reactive oxygen species (ROS) induces the generation of danger signals to activate the innate immune response. Such a ROS-mediated OS can be detrimental to the survival of cell carriers as well as to their ability to carry the therapeutic cargo to distant targeted sites. *N*-acetylcysteine (NAC) is a widely used low-molecular weight thiol antioxidant. The neutralization of the carboxylic group of NAC generated a more lipophilic and cell-permeable component, *N*-acetylcysteine amide (NACA).

Experiments have been carried out to determine the therapeutic effects of the combination of NACA and CRAd-S-pk7 OV loaded NSCs on glioma progression. The *in vitro* results indicate that NACA treatment combined with CRAd-S-pk7 loaded HB1.F3.CD NSCs increases the production of viral progeny and significantly enhances glioma cell oncolysis compared to CRAd-S-pk7 loaded NSCs alone. It was also found that NACA decreases viral-induced levels of intracellular ROS and prevents CRAd-S-pk7 induced apoptosis of NSCs. Furthermore, it was observed that the combined treatment increases virus production in the stem cell carrier and enhances apoptosis of glioma tissues, which leads to a higher animal survival. NACA appears to be a valid partner for a combination with CRAd-S-pk7 loaded NSCs in anti-glioma therapy.

### Materials and Methods

### Cell culture, antibodies and viruses

U87 (purchased from the American Tissue Culture Collection) and U87-Luciferase-neomycin (U87-LucNeo) cells were maintained in Dulbecco's modified Eagle's medium (DMEM) supplemented with 10% FBS and 100 units of penicillin/streptomycin at 37°C with 5% CO₂. HB1.F3.CD is a *v*-*myc* immortalized human NSC (hNSC) line, derived from the human fetal brain that constitutively expresses cytosine deaminase (CD). HB1.F3.CD cells were maintained in DMEM supplemented with 10% fetal bovine serum, 2 mmol/L L-glutamine, 100 units/mL penicillin, 100 ug/mL streptomycin and 0.25 ug/mL amphotericin B. Anti-p53 (DO-1), actin and active caspase-3 antibodies were purchased from Santa Cruz biotechnology. Anti phospho-Akt, phospho-p38 and caspase-9 antibodies were purchased from Cell Signaling Technology. Anti-Hexon antibody was purchased from the Abcam. Human CRAd-Survivin-pk7 (CRAd-S-pk7) was propagated as described previously (5, 37), and NACA was provided by Dr. Glenn Goldstein (David Pharmaceuticals, New York, NY).

### Cell viability assay

HB1.F3.CD and glioma cells were infected with CRAd-S-pk7 (50 I.U./cells) for 1 hour followed by washing with PBS twice. Infected cells (5 × 10³ cells/well) were plated onto a 96 well plate and further incubated using the complete medium with/without NACA (1 mM) in a dose- and time- dependent manner. Ten µl of 3-(4,5-dimethylthiazol-2-yl)-2,5-diphenyltetrazolium bromide was added into each well followed by incubation at 37 °C for 4 h. Finally, 100 µl of solubilization buffer was added. The plate was incubated at 37 °C overnight, and light absorption was measured at a 595 nm wavelength. Cell viability was also evaluated by trypan blue exclusion method using the Bio-Rad automated cell counter (TC10). Each value from these indicated assays represents the mean ± SEM of triplicate measurements from three independent experiments.

### Analysis of viral replication and progeny

For quantitative PCR analysis of adenoviral E1A gene expression, HB1.F3.CD cells (5 × 10⁴ cell/well) were plated in 6 well culture dishes. On the next day, plated cells were infected with CRAd-S-pk7 at the indicated infection unit per cell for 1 hour and then carefully washed with PBS following the addition of fresh complete media with/without NACA. Total DNA from cultured cells (or animal brain tissue) was extracted at designated time points using DNeasy Tissue kit (QIAGEN) according to the manufacturer's protocol. DNA was quantified using NANO drop and was subsequently used for quantitative real-time PCR with iQ SYBR green supermix (Bio-Rad, Hercules, CA). Sequences of primers for detection of the E1A gene were the following: forward, 5'-AACCAGTTGCCGTGAGAGTTG-3' (SEQ ID NO:1); and reverse, 5'-CTCGTTAAGCAAGTCCTCGATACAT-3' (SEQ ID NO:2). DNA amplification was carried out using an Opticon 2 system (Bio-rad, CA). All samples were run in triplicates and results were shown as the average number of E1A copies/ng of DNA.

To investigate the production of viral progeny in NSCs infected with CRAd-S-pk7 and then treated with or without NACA, HB1.F3.CD cells were seeded at a concentration of 50,000 cells per well in a 6 well plate using DMEM containing 10% FBS. Twenty-four hours after incubation, cells were infected with CRAd-S-pk7 at several infection units per cell and further incubated for 1 hour. Then virus-containing media were removed, cells were washed with 1X PBS and fresh complete media with/without NACA was added. After incubation for an indicated time, supernatant as well as cells were collected separately. Collected cells were suspended in 200 µl of 1X PBS and were lysed by freezing and thawing three times. Lysed cells were incubated in 80% confluent HEK293 cells at a serial ten-fold dilution condition. Forty-eight hours after incubation, viral titer (infection units per milliliter) was determined by Adeno-X Rapid Titer Kit (Clontech, CA) according to the manufacturer's protocol.

### Analysis of glioma cell cytotoxicity by viral progeny released from NSCs

To evaluate the cytotoxic effect of oncolytic viruses released from NSCs toward glioma cells, HB1.F3.CD cells (5 × 10⁴ cell/well) were plated in 6 well culture dishes. Cells were infected with CRAd-S-pk7 (50 I.U./cell). One hour after incubation, cells were carefully washed with pre-warmed 1X PBS twice and then fresh complete media with/without 1 mM NACA was added. After 4 days, supernatant and infected U87 cells (5 × 10⁴ cell/well in 6 well plates) were secured. The viability of glioma cells was determined by 0.25% crystal violet staining for 20 minutes at room temperature. Stained cells were visualized with an inverted microscope and counted (20X magnification).

### Luciferase assay (co-culture)

For determination of virus-mediated cytotoxicity, HB1.F3.CD cells infected with CRAd-S-pk7 were plated in 96 well culture plates at a cell density of 2,500 (U87-Luc and HB1.F3.CD cell ratio=1:0.5) or 5,000 (U87-Luc and HB1.F3.CD cell ratio=1:1) cells/well and incubated in a humidified incubator at 37°C with 5% CO₂. CRAd-S-pk7-permissive U87 cells were used as a positive control for adenoviral replication and virus induced cytopathic effect. Four hours later U87-Luc cells (5× 10³ cells per well) were plated in HB1.F3.CD cultured plates and further incubated for 6 days. Luciferase activity was performed using Dual-Luciferase Reporter Assay System (Promega) according to the provided protocol. Corresponding firefly luciferase reporter activity was used for normalization.

### Determination of Reactive Oxygen Species (ROS) Generation in NSCs

Endogenous ROS generation in NSCs was accessed by staining cells with 5-(and-6) chlolomethyl-2',7'-dichlorfluorescein-diacetate (CM-H₂DCFDA; Molecular Probes). Briefly, HB1.F3.CD cells were incubated in 6 well plates for 24 hours. After infecting cells with or without CRAd-S-pk7 (10 or 50 I.U) for 1 hour, cells were washed with fresh 1X PBS twice followed by the addition of complete media with/without 1 mM NACA. After 48 hours, cells were incubated with 10 µM CM- H₂DCFDA in serum free media at 37°C for 30 minutes according to manufacturer's instruction. H₂O₂ treatment was used as a positive control. Cells were treated with 50 µM H₂O₂ before staining with CM-H₂DCFDA. Subsequently, cells were fixed with 2% paraformaldehyde, and images were captured with fluorescence microscope. Three fields from each of three separate wells were measured for each group. Fluorescence was quantified from 30 random cells in each image using NIH image J software.

### Western blot analysis

HB1.F3.CD cells were incubated with various concentrations of CRAd-S-pk7 and/or NACA. Cells were washed and harvested in 1X PBS and subsequently lysed with lysis buffer (M-PER Mammalian Protein Extraction Reagent (Pierce) supplemented with 10mM Protease and Phosphatase Inhibitor cocktail (Roche)) according to the manufacturer's instructions. Thirty µg of each cell lysate was used for SDS-PAGE electrophoresis and immunoblotting. Immunocomplexes were visualized with enhanced chemiluminescence reagent (Bio-Rad). Images were collected using a Bio-Rad image analyzer.

### Animal experiments

The University of Chicago Institutional Animal Care and Use Committee approved the animal studies and all procedures. The anti-glioma therapeutic efficacy of the combination of OV-loaded NSCs and NACA was determined in an *in vivo* intracranial animal model. Briefly, 6 week old athymic nude mice were housed in laminar-flow cabinets under specific-pathogen-free conditions. Mice were anesthetized by intraperitoneal (i.p.) injection with a ketamine/xylazine mixture (115/17 mg/kg), and were held in a stereotactic frame with an ear bar. After skin incision, a burr hole was made in the skull 1 mm anterior and 2 mm lateral to the bregma to expose the dura. U87 cells (2 × 10⁵ cells in a volume of 2.5µl of PBS) were slowly injected 3 mm deep into the brain with a Hamilton syringe. Four days after tumor implantation, the animals were randomly divided into four groups, and were intraperitoneally treated with NACA (250 mg/kg/day) continuously for 5 days. On the next day after NACA first treatment, mice received an intracranial or intratumoral injection of 4 × 10⁵ HB1.F3.CD cells loaded with 50 I.U. of CRAd-S-pk7 oncolytic viruses. The four groups were: group 1 (PBS i.p,, control, *n*=6), group 2 (NACA, 250mg/kg i.p., *n*=6), group 3 (HB1.F3.CD cells loaded with CRAd-S-pk7 50 I.U., *n*=7) and group 4 (NACA, 250 mg/kg i.p. plus HB1.F3.CD cells loaded with CRAd-S-pk7 50 I.U., *n*=7). Neurological signs and body weight were checked every day during the treatment. Animals losing ≥30% of their body weight or having trouble ambulating, feeding, or grooming were killed by CO₂ intoxication followed by cervical dislocation and the brains were removed. Midcoronal sections of the whole tumor were used for histological and immunohistochemical analyses.

### Analysis of immunohistochemistry staining

Tumors were dissected from the sacrificed mice, fixed in 10% buffered formalin solution, and frozen in OCT compound in a dry ice-methylbutane bath. Frozen tissues were cut coronally at the injection site in two pieces and sectioned into 10 µm thick slices. Immunofluorescent stains were applied using monoclonal antibodies against caspase 3 and E1A. Brain sections were dry at room temperature and fixation/permeabilization was carried with 50/50 mixture of acetone-methanol. The tissues were three times washed with cold PBS and blocked with 10% BSA for 30 minutes. Samples were incubated overnight at 4°C with primary antibodies, washed and incubated for 1 hour at room temperature with the secondary antibody. After washing with cold PBS three times, tissues were covered with Prolong Gold antifade reagent with DAPI (Invitrogen). Images were acquired using an inverted Zeiss microscope.

### Statistical Analysis

Experimental data were analyzed for statistical significance using SigmaPlot, statistical analysis software (Systat Software) and GraphPad Prism 4 (GraphPad Software Inc., San Diego CA). All experiments were performed in a blinded manner. Survival curves were generated by Kaplan-Meier method, and log-rank test was used to compare the distributions of survival times. The data were expressed as mean ± SEM. Statistical significance was defined as *, P < 0.05 and **, P < 0.001.

### Results

### NACA improves the viability of NSC carriers loaded with CRAd-S-pk7

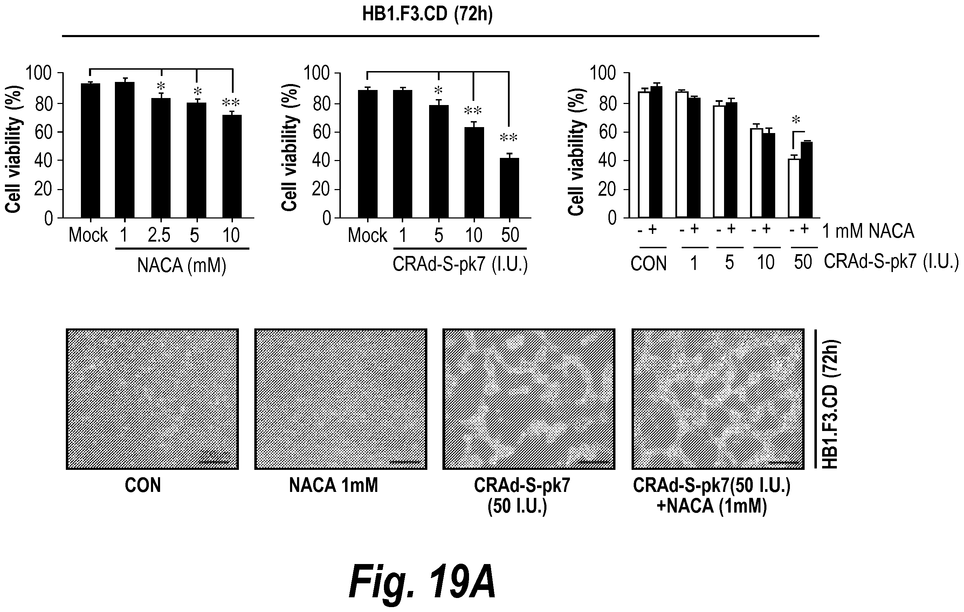

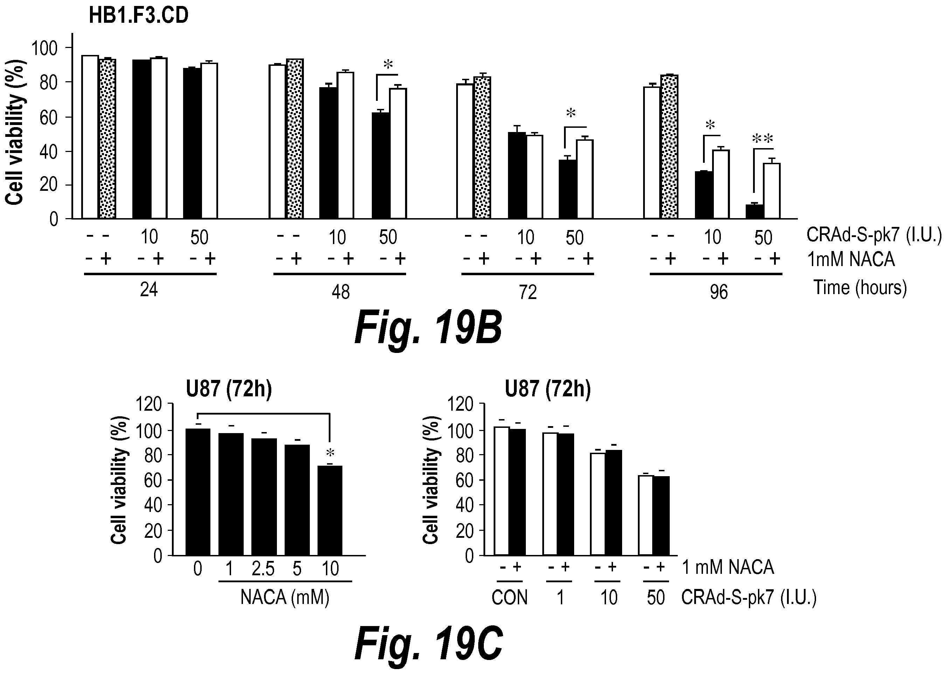

HB1.F3.CD NSC carriers were permissive for CRAd-S-pk7 infection and replication. It appears that the antioxidant effects of NACA inhibit cellular apoptosis and thus increase the intracellular production of CRAd-S-pk7 loaded in HB1.F3.CD stem cell carriers. To test the anti-apoptotic effects of NACA on OV-loaded NSCs, the effect of NACA combined with CRAd-S-pk7 on the viability of both NSCs and glioma cells was assessed. To measure NACA-related cytotoxicity, an MTT assay and trypan blue exclusion test were used at 72 hours post treatment. The results revealed that NACA was toxic to NSCs at concentrations over 1 mM (Fig. 19A), whereas toxicity was observed in U87 cells at a concentration of 10 mM (Fig. 19C). The ImM concentration showed to be optimal because it allowed evaluation of the production of infectious adenoviral progeny without affecting the viability of NSCs. Therefore, the concentration of 1 mM of NACA was chosen to be used in our further *in vitro* experiments. CRAd-S-pk7 was toxic to HB1.F3.CD cells in a dose dependent fashion. As shown in Figure 19A, viability of the NSCs was reduced by 13% at the dose of 5 I.U./cell, 27% at 10 I.U/cell and about 53% when NSCs were infected by 50 I.U at 72 hours post-infection. An increased viability of NSCs was observed when 50 I.U. of CRAd-S-pk7 was combined with 1 mM of NACA as compared to other combinations (1, 5 and 10 I.U of OV per cell). NACA combined with CRAd-S-pk7 (50 I.U.) increased the viability of NSCs 48 hours post-treatment. Moreover, treatment with 10 I.U of CRAd-S-pk7 and NACA showed an enhanced viability of NSC carriers 96 hours post-treatment as compared to CRAd-S-pk7 loaded NSCs alone (Fig. 19B).

To further evaluate the effects of NACA combination with OVs on glioma cell viability, we infected U87 cells with various concentrations (1, 10 and 50 I.U./cell) of CRAd-S-pk7 and measured the viability of cells after treatment with NACA via trypan blue exclusion. In contrast with NSCs, the combination of NACA and OV did not show an enhanced viability of U87 cells (Fig. 19C). Therefore, the data suggest that low-dose NACA, which has no cytotoxic effect in NSCs, enhances the viability of OV loaded NSCs and do not increase the viability of U87 cells.

### NACA treatment on CRAd-S-pk7 loaded NSCs increases viral replication and production of viral progeny

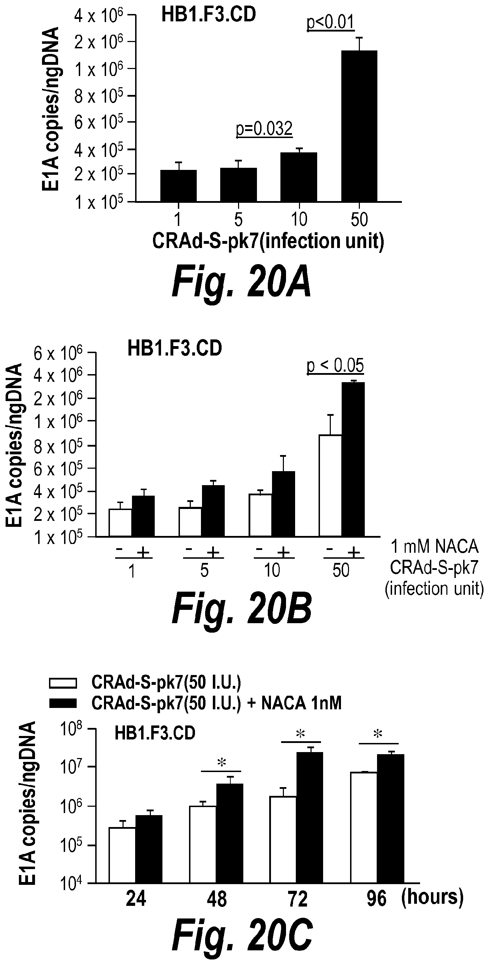

DNA replication of CRAd-S-pk7 in stem cell carriers was highest at day 3 when cells were infected with 50 I.U/cell of OVs. Although, virus replication reached its peak, the viability of NSCs was decreased at this dose. Thus, in order to determine the replication kinetics of CRAd-S-pk7 in NSCs treated with NACA, quantitative RT-PCR analysis was conducted to check the expression of the adenoviral E1A gene at 72 hours post treatment. As shown in Figure 20, when compared to the infection of OV alone, viral DNA replication was significantly increased when NSCs loaded with OV in the concentration of 50 I.U/cell (1.78 × 10⁶ E1A copies/ng DNA) were treated with NACA. Combination of 50 I.U of OV loaded into NSCs treated with 1 mM of NACA showed a 4.2 fold increase in viral replication compared to OV single treatment (*p*=0.024) (Fig. 20B). Contrarily, other combinations with various concentrations of CRAd-S-pk7 (1, 5, and 10 I.U/cell) and ImM of NACA showed little improvement of viral replication in NSCs as compared to single treatment (p > 0.05). Time course treatment of CRAd-S-pk7 with ImM of NACA demonstrated that virus replication had significantly increased from 48 to 96 hours post treatment as compared to OV single treatment. The highest effect (about a log) was observed at 72 hours post-initial therapy (Fig. 20C).

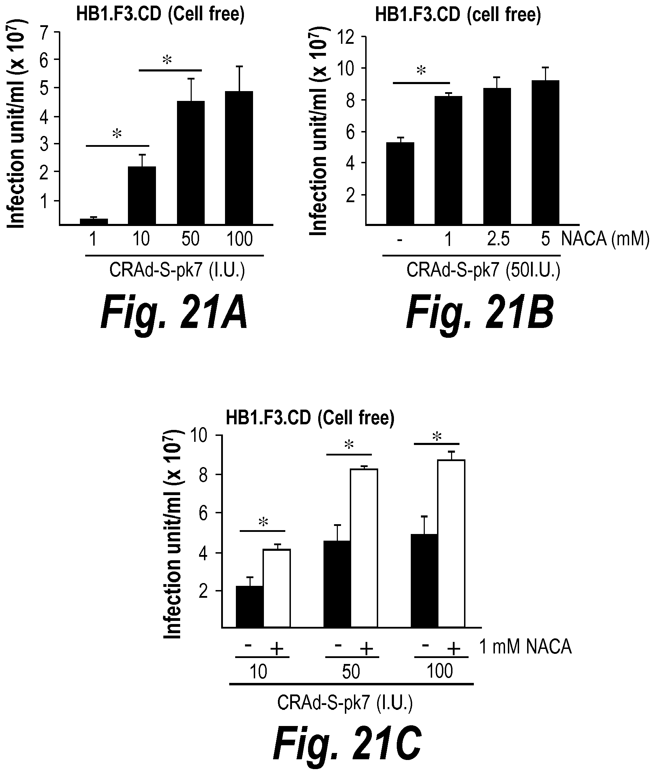

To evaluate viral production in NSC carriers treated with NACA, a viral titer assay was performed. The production of CRAd-S-pk7 viral progeny in HB1.F3.CD cells increased in a dose-dependent manner. At 50 I.U. of CRAd-S-pk7 per cell, the viral production was about 2-folds higher than in the infection dose of 1 and 10 I.U/cell, respectively (Fig. 21A). However, CRAd-S-pk7 infection in the concentration of 100 I.U./cell did not show an enhanced viral production as compared to 50 I.U./cell. Then, viral release from NSCs treated with various concentrations of NACA (1, 2.5, 5 mM) but a single concentration of OV (50 I.U./cell) was assessed. NSCs were harvested and subjected to total viral titer evaluation after 3 days of incubation. As a result, NSCs loaded with 50 I.U/cell of OVs treated with ImM of NACA presented a significant increase (61%) in OV release as compared to untreated carriers (Fig. 21B). NSCs treated with 2.5 and 5 mM of NACA did not present a significant viral release as compared to NSCs treated with 1 mM of NACA.

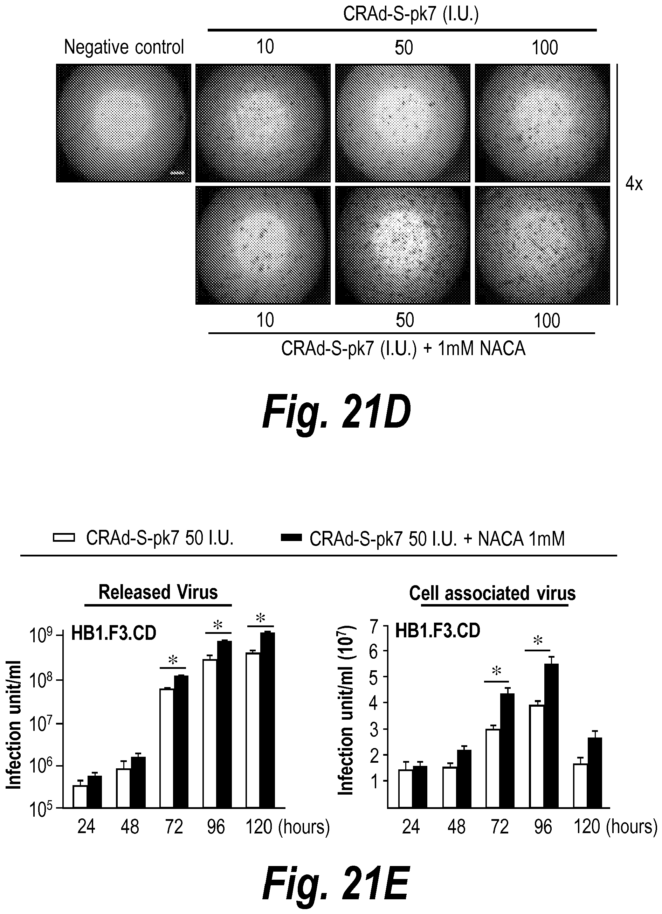

The release of OV progeny in NSCs treated with or without ImM of NACA and loaded with different doses of CRAd-S-pk7 OVs (10, 50 and 100 I.U./cell) was then assessed. The supernatant of infected cells was harvested 3 days post-infection. HB1 .F3.CD cells released a significant amount of viruses from each combination of OV and NACA. Such a discharge was about 2-2.5 fold larger than the one observed in cells infected with OV alone (Figs. 21C and D). In order to determine replication and viral release in a in time dependent fashion, NSCs were infected with 50 I.U/cell of CRAd-S-pk7 and harvested cells and supernatant separately at indicated times. As shown in Figure 21E, the combination of OV and NACA showed additive effects in both groups of released virus and cell associated virus as compared to OV only after 72 hours post infection. Free viruses that were collected in the supernatant of NSCs treated with NACA maintained an active replication until day 5 post-collection. In the cell associated virus group (NSCs infected with OVs that were collected apart from the supernatant), virus replication was higher at 96 hours post treatment, and then it significantly decreased at day 5 due to cell death resultant from viral toxicity. Taken together, these data indicate that NACA significantly induces virus replication in NSCs loaded with oncolytic vectors and enhances the release of their associated viral progeny.

### NACA treatment of NSCs loaded with CRAd-S-pk7 leads to increased production of infectious viral progeny and induction of glioma cell oncolysis.

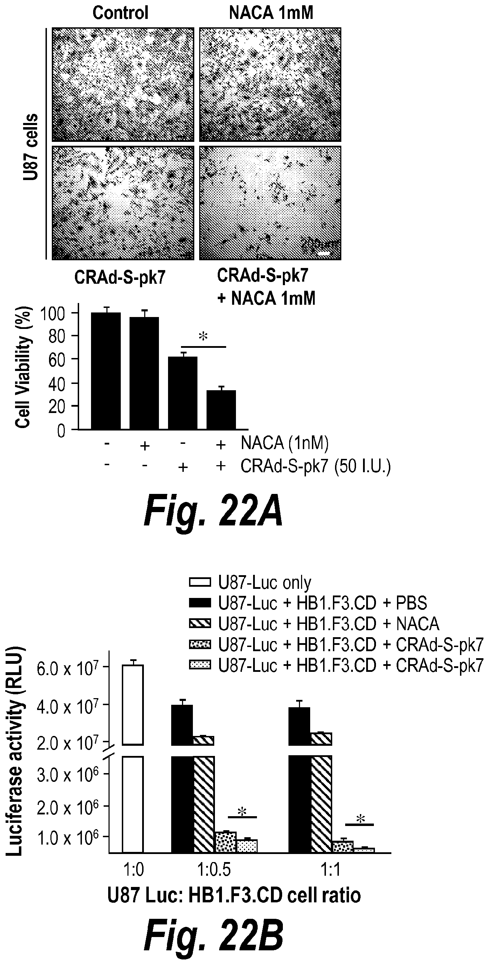

Next, the capacity of the viral progeny released from NSCs to lyse targeted tumor cells was examined. Previously, it was shown that NSCs were permissive to CRAd-S-pk7 infection and replication. To assess if NACA treatment would affect the release of viral progeny from NSCs loaded with OVs and its related toxicity to glioma cells, the supernatant containing the viral progeny released from infected NSCs treated or not with NACA was collected. Then trypan blue exclusion and crystal violet assay were used to measure CRAd-S-pk7-related toxicity towards U87 glioma cells 96 hours post-infection. Figure 22A brings a representative image demonstrating U87-related cytotoxicity as a result of CRAd-S-pk7 infection alone or combined with NACA treatment. Here, U87 cells treated with NACA alone and U87 mock cells were used as a control. As shown in Figure 22A, CRAd-S-pk7 viral progeny treated with NACA promote a significantly higher lysis of glioma cells (73% toxicity) as compared to treatment with CRAd-S-pk7 alone (38% toxicity). To further assess the cytotoxic effects of OV therapy combined or not with NACA on glioma cell viability, a co-culture experiment was performed using U87 cells tagged with Luciferase report (U87-Luc) and HB1.F3.CD NSCs loaded with CRAd-S-pk7 OVs. First, NSCs loaded with CRAd-S-pk7 (50 I.U./cell) were plated in a 96 well plate in two concentrations: 2500 and 5000 cells/well. Then, U87-Luc cells were plated onto the pre-cultured wells following respectively the concentrations of 1 U87:0.5 NSCs and 1 U87:1 NSC. Co-cultured cells in both conditions were incubated for 6 days, when glioma cells were collected, lysed and luciferase activity was measured by luciferase assay (Promega). A decreased luciferase activity was found in U87-Luc cells infected with CRAd-S-pk7 treated with NACA as compared to infected U87-Luc cells that did not receive NACA (Fig. 22B). Thus, U87-Luc cells infected with CRAd-S-pk7 presented a higher cytotoxicity when compared to its control without NACA. Moreover, non-infected U87 cells treated with NACA presented lower rates of cell lysis as compared to its mock control (U87 cells alone). Taken togeher, the above described results demonstrate that NACA may effectively improve the therapeutic efficacy of oncolytic virotherapy and thereby enhance glioma oncolysis.

### NACA modulates endogenous ROS and decreases the expression of markers of cellular apoptosis in NSCs infected with CRAd-S-pk7.

Previous studies have demonstrated that the generation of reactive oxygen species (ROS) was closely related to the activation of proinflammatory pathways that happened as a response to viral infection. Other reports have also correlated the generation of ROS with the promotion of cellular transformation and tumorigenesis through DNA oxidation and subsequent gene mutation. Recently, a couple of additional studies have shown that cellular infection with OVs resulted in the accumulation of endogenous ROS in microglia and monocytic leukemia cells. Such an accumulation was able to induce the expression of proinflammatory cytokines and mitochondrial dysfunction in the host cell. Based on these findings, the mechanisms underlying OV-mediated ROS accumulation in NSC carriers were investigated. To answer this question, it was first checked if OV infection would be able to affect endogenous ROS levels in stem cell carriers. As shown in Figure 23A, HB1.F3.CD cells infected with CRAd-S-pk7 presented ROS accumulation in a dose-dependent manner. The combined treatment with NACA, however, effectively decreased endogenous ROS levels that were activated by cellular loading with oncolytic viruses. Further, non-infected NSCs that were used as a control inherently expressed high levels of intracellular ROS. Such overexpression was decreased by NACA treatment.

It has been previously shown that ROS accumulation in NSCs can lead to a number of different outcomes, such as decreased cellular proliferation and H₂O₂-induced apoptosis. Thus, NACA's anti-apoptotic and pro-proliferative effects on NSCs loaded with OVs were examined by immunoblot assay. NSCs loaded with OVs presented an increased expression of pro-apoptotic genes (p53 and caspase-3) in a dose-dependent manner (Fig. 23B). Infected NSCs treated with NACA, however, presented a significant decrease in p53 and caspase-3 expression. In connection with the signaling pathways related to pro-apoptotic stimuli, a recent report showed that ROS-dependent activation of Akt, Erkl/2 and p38 mitogen-activated protein kinases in NSCs was associated with decreased self-renewal and proliferation. Based on this information, It was investigated if NACA treatment was able to prevent OV-induced increase in the intracellular levels of ROS through inhibition of p-Akt and p-p38 signaling pathways. NSCs loaded with CRAd-S-pk7 treated with NACA presented decreased activation of Akt and p38 pathways and reduced expression of apoptosis-related genes (p53, caspase-9 and caspase-3) (Figs. 23C and D). We also found that the combination of CRAd-S-pk7 and NACA induced a higher expression of the adenovirus late protein (Hexon) as compared to OV alone. Hence, the results suggest that NACA effectively reduces the levels of apoptosis-related proteins as well as downregulates PI3K (phosphatidylinositide 3-kinase) and MAPK (mitogen-activated protein kinase) signaling pathways in CRAd-S-pk7 loaded NSCs through modulation of intracellular ROS.

### The combination therapy of NACA and CRAd-S-pk7 loaded NSCs prolongs mice survival in an orthotopic glioma model.

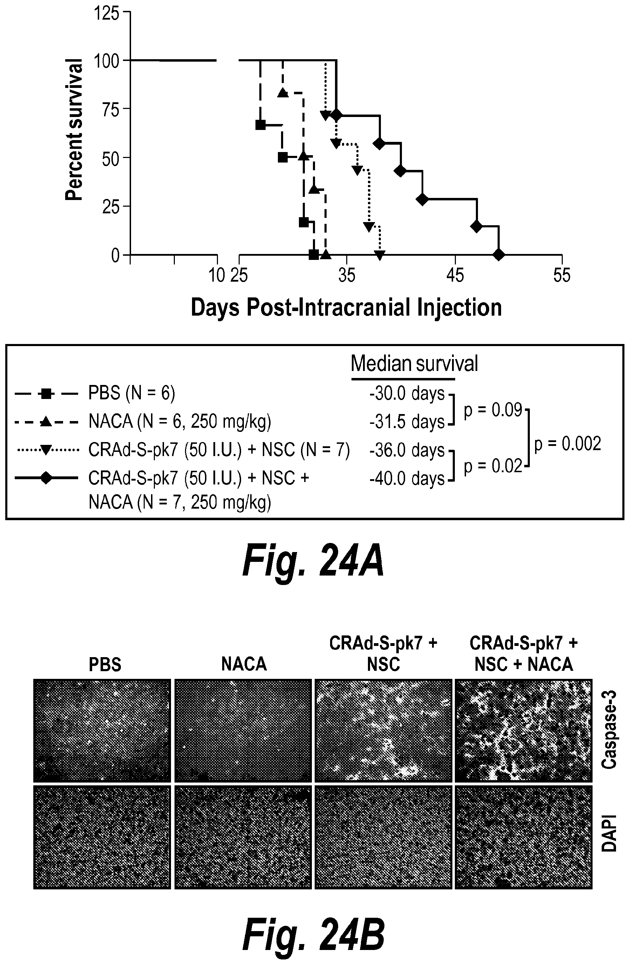

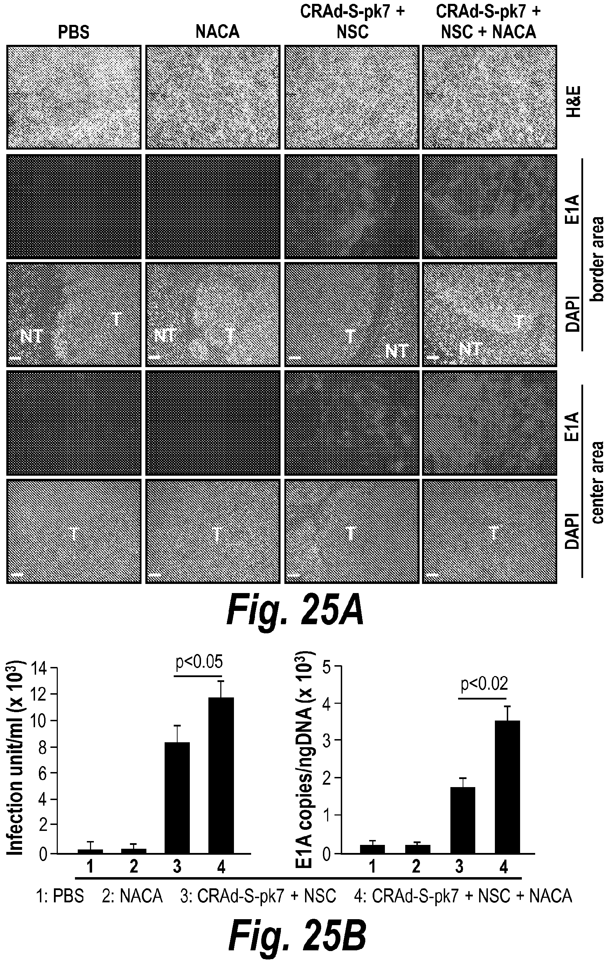

As a lipophilic drug, NACA has the striking advantage of crossing the blood brain barrier (BBB). It has previously shown that NSCs could be used as delivery vehicles in order to enhance the anti-glioma therapeutic efficacy of OVs *in vivo.* Therefore, whether NACA treatment was able to increase the therapeutic efficacy of NSCs loaded with CRAd-S-pk7 OVs in a glioma xenograft model was evaluated. U87 cells were implanted in the brain of nude mice. Four days after tumor implantation, mice were divided into four groups: (1) control group, without treatment; (2) NACA alone group; (3) NSCs loaded with OV group; and (4) NSCs loaded with OV in mice systemically treated with NACA. Groups 2 and 4 received NACA intraperitoneally four days post-tumor implantation, in the concentration of 250 mg/kg/day for 5 days. Groups 3 and 4 received NSCs (4 × 10⁵ cells/animal) loaded with CRAd-S-pk7 (50 I.U/cell) intratumorally also five days after tumor implantation. Mice treated with the combination of NACA and NSCs loaded with OVs presented a significantly higher survival (median survival of 40 days) than mice treated with NSCs loaded with OVs alone (median survival of 36 days) (Fig. 24A). NACA treatment alone, however, did not improve animal survival (median survival of 31.5 days) as compared to the PBS control group (median survival of 30 days). An important finding was that tumors treated with the combination of NACA and NSCs loaded with CRAd-S-pk7 presented an increased expression of pro-apoptotic markers such as caspase-3 as compared to NSCs loaded with OV alone (Fig. 24B). One of the main advantages of using NSCs loaded with OVs is their ability of penetrate the tumor and reach infiltrative neoplastic areas. To evaluate if NACA treatment would affect NSC migration and intratumoral viral distribution *in vivo,* the expression of an early-phase protein that drives viral genome replication (E1A) was examined in tumor sections. Mice were sacrificed 21 days post tumor implantation, their brains were collected and subjected to immunohistochemical analysis using adenoviral E1A antibody. As shown Figure 25A, tumors treated with the combination of NACA and NSCs loaded with CRAd-S-pk7 showed a significant increase in the expression of E1A viral protein as compared to mice treated with NSCs loaded with CRAd-S-pk7 alone. Also, viral E1A protein was widely spread throughout the tumor in the combination group (group 4) as compared to the NSCs plus CRAd-S-pk7 group (group 3). On the second repeat of the experiment the mice brains were collected and dissociated. Then, quantitative RT-PCR analysis was used to assess the amount of viral DNA and viral progeny *in vivo.* A significant increase in CRAd-S-pk7 viral E1A and progeny in the NACA combination group was found as compared to the OV loaded NSCs group (Fig. 25B). Taken together, these data indicate that NACA enhances the therapeutic efficacy of CRAd-S-pk7 loaded NSCs by increasing intratumoral apoptosis and improving viral distribution in intracranial glioma xenografts.

### Discussion

Despite the existence of advanced therapeutic strategies to treat GBM patients, their mean overall survival has little improved over the past decades. Also, the currently available treatment options that are effective for GBM patients are extremely limited. Therefore, it is undeniable that these patients are in need of novel and more effective therapeutic approaches. Oncolytic virotherapy using NSCs as carriers is one of the most promising areas of advancement.

A number of studies have shown that anti-glioma oncolytic virotherapy has a prominent potential. However, the application of such strategy in clinical trials is still limited due to elevated production of cytokines such as IL-6 and TNF-α that elicit the innate immune response against viral infection.

In general, systemically administered OVs are quickly eliminated by the innate immune response before they are able to reach the target tumor site. Previous reports demonstrated that NSCs not only have the capacity to migrate towards tumor areas, but they also possess immunosuppressive properties. Regarding the ability of NSCs to deliver OVs, we have reported that NSCs loaded with OVs were able to increase viral distribution, suppress anti-viral innate immune response, and extend animal median survival by 50% as compared to oncolytic virotherapy alone. Although only 20-30% of the NSCs contralaterally injected in the mouse brain were able to migrate to the targeted glioma area, the therapeutic effect was considered significant. In order to investigate if NACA treatment could increase the *in vivo* viability of NSC carriers, enhance viral replication and improve therapeutic efficacy, an *in vivo* analysis of the anti-tumor effects of the combination of NACA plus NSCs loaded with OVs on the survival of mice bearing intracranial gliomas was performed. It was found that NACA combination therapy significantly prolonged mice overall survival and promoted even intratumoral viral distribution as compared to NSCs loaded with OVs alone.

### Reference Example 6: NACA for the treatment of asphyxia

This is outline of current study which tests for protection from asphyxia and blocking brain cell and nerve damage as a result of instances of lack of oxygen in all tissues. The results are also applicable to support use of NACA for blocking reperfusion injury.

Piglets of 12-36 hrs of age are given anesthesia. The piglets are orally intubated on a ventilator and central vein access is provided. After one hour of stabilization the following is performed on randomized groups.
Controls (n=6)
Intervention group (N = 4 x 10):
Global hypoxia breathing 8% O₂ in N₂. CO₂ will be added during hypoxemia aiming at a pCO2 of 8.0-9.5 kPa (to imitate perinatal asphyxia). When MABP decrease to 20 mmHg or BE <-20, piglets will be randomized to 4 different treatment groups.
Group 1 will be resuscitated with 21% oxygen and receive NACA (shortly after start of resuscitation and thereafter a new dose after 5h).
Group 2 will be resuscitated with ambient air and not receive NACA
Group 3 will be resuscitated for 30 min with 100% oxygen and receive NACA (shortly after start of resuscitation and thereafter a new dose after 5h).
Group 4 will be resuscitated for 30 min with 100% oxygen and will not receive NACA.
The piglets will be observed for 9.5 h after the end of hypoxia.
At the end of the observation time, the piglets will be given an overdose of Pentobarbital (150mg/kg). The total time of the experiment, from induction of anesthesia to the end, will be approximately 12.5h.

### REFERENCES

Patel, S. P., Sullivan P.G., Lyttle T.S., Magnuson, D. S. K. and Rabchevsky A.G., (2012) Acetyl-1-carnitine treatment following spinal cord injury improves mitochondrial function correlated with remarkable tissue sparing and functional recovery. (In Revision) Neuroscience*.*
Aghi M, Martuza RL. 2005. Oncolytic viral therapies - the clinical experience. Oncogene 24:7802-7816.
Ahmed AU, Rolle CE, Tyler MA, Han Y, Sengupta S, Wainwright DA, Balyasnikova IV, UlasovIV, Lesniak MS. 2010. Bone marrow mesenchymal stem cells loaded with an oncolytic adenovirus suppress the anti-adenoviral immune response in the cotton rat model. Mol Ther 18:1846-1856.
Ahmed AU, Thaci B, Alexiades NG, Han Y, Qian S, Liu F, Balyasnikova IV, Ulasov IY, Aboody KS, Lesniak MS. 2011. Neural stem cell-based cell carriers enhance therapeutic efficacy of an oncolytic adenovirus in an orthotopic mouse model of human glioblastoma. Mol Ther 19:1714-1726.
Ahmed AU, Tyler MA, Thaci B, Alexiades NG, Han Y, Ulasov IV, Lesniak MS. 2011. A comparative study of neural and mesenchymal stem cell-based carriers for oncolytic adenovirus in a model of malignant glioma. Mol Pharm 8:1559-1572.
Ahmed AU, Ulasov IV, Mercer RW, Lesniak MS. 2012. Maintaining and Loading Neural Stem Cells for Delivery of Oncolytic Adenovirus to Brain Tumors. Methods Mol Biol 797:97-109.
Benhar M, Dalyot I, Engelberg D, Levitzki A. 2001. Enhanced ROS production in oncogenically transformed cells potentiates c-Jun N-terminal kinase and p38 mitogen-activated protein kinase activation and sensitization to genotoxic stress. Mol Cell Biol 21 :6913-6926.
Cheema TA, Kanai R, Kim GW, Wakimoto H, Passer B, Rabkin SD, Martuza RL. 2011. Enhanced antitumor efficacy of low-dose Etoposide with oncolytic herpes simplex virus in human glioblastoma stem cell xenografts. Clin Cancer Res 17:7383-7393.
DeNicola GM, Karreth FA, Humpton TJ, Gopinathan A, Wei C, Frese K, Mangal D, Yu KH, Yeo CJ, Calhoun ES, Scrimieri F, Winter JM, Hruban RH, Iacobuzio-Donahue C, Kern SE, Blair IA, Tuveson DA. 2011. Oncogene-induced Nrf2 transcription promotes ROS detoxification and tumorigenesis. Nature 475:106-109.
Fink K, Duval A, Martel A, Soucy-Faulkner A, Grandvaux N.. 2008. Dual role of NOX2 in respiratory syncytial virus- and sendai virus-induced activation of NF-kappaB in airway epithelial cells. J Immunol 180:6911-6922.
Fisher K. 2006. Striking out at disseminated metastases: the systemic delivery of oncolytic viruses. Curr Opin Mol Ther 8:301-313.
Giese A, Westphal M. 1996. Glioma invasion in the central nervous system. Neurosurgery 39:235-250; discussion 250-232.
Glass R, Synowitz M, Kronenberg G, Walzlein JH, Markovic DS, Wang LP, Gast D, Kiwit J, Kempermann G, Kettenmann H. 2005. Glioblastoma-induced attraction of endogenous neural precursor cells is associated with improved survival. J Neurosci 25:2637-2646.
Gong X, Celsi G, Carlsson K, Norgren S, Chen M. 2010. N-acetylcysteine amide protects renal proximal tubular epithelial cells against iohexol-induced apoptosis by blocking p38 MAPK and iNOS signaling. Am J Nephrol 31:178-188.
Grinberg L, Fibach E, Amer J, Atlas D. 2005. N-acetylcysteine amide, a novel cell-permeating thiol, restores cellular glutathione and protects human red blood cells from oxidative stress. Free Radic Biol Med 38:136-145.
Hockenbery DM, Oltvai ZN, Yin XM, Milliman CL, Korsmeyer SJ. 1993. Bcl-2 functions in an antioxidant pathway to prevent apoptosis. Cell 75:241-251.
Hu S, Sheng WS, Schachtele SJ, Lokensgard JR. 2011. Reactive oxygen species drive herpes simplex virus (HSV)-1-induced proinflammatory cytokine production by murine microglia. J neuroinflammation 8:123.
Kim J, Wong PK. 2009. Loss of ATM impairs proliferation of neural stem cells through oxidative stress-mediated p38 MAPK signaling. Stem Cells 27:1987-1998.
Kongara S, Karantza V. 2012. The interplay between autophagy and ROS in tumorigenesis. Front Oncol 2:171.
Kornblith PK, Welch WC, Bradley MK. 1993. The future of therapy for glioblastoma. Surg Neurol 39:538-543.
Krex D, Klink B, Hartmann C, von Deimling A, Pietsch T, Simon M, Sabel M, Steinbach JP, Heese O, Reifenberger G, Weller M, Schackert. 2007. Long-term survival with glioblastoma multiforme. Brain 130:2596-2606.
Lorence RM, Rood PA, Kelley KW. 1988. Newcastle disease virus as an antineoplastic agent: induction of tumor necrosis factor-alpha and augmentation of its cytotoxicity. J Natl Cancer Inst 80:1305-1312.
Madhavan L, Ourednik V, Ourednik J. 2006. Increased "vigilance" of antioxidant mechanisms in neural stem cells potentiates their capability to resist oxidative stress. Stem Cells 24:2110-2119.
Martin V, Herrera F, Garcia-Santos G, Antolin I, Rodriguez-Blanco J, Rodriguez C. 2007. Signaling pathways involved in antioxidant control of glioma cell proliferation. Free Radic Biol Med 42:1715-1722.
McGuire KA, Barlan AU, Griffin TM, Wiethoff CM. 2011. Adenovirus type 5 rupture of lysosomes leads to cathepsin B-dependent mitochondrial stress and production of reactive oxygen species. J Virol 85:10806-10813.
Nicholas MK, Lukas RV, Chmura S, Yamini B, Lesniak M, Pytel P. 2011. Molecular heterogeneity in glioblastoma: therapeutic opportunities and challenges. Semin Oncol 38:243-253.
Offen D, Gilgun-Sherki Y, Barhum Y, Benhar M, Grinberg L, Reich R, Melamed E, Atlas D. 2004. A low molecular weight copper chelator crosses the blood-brain barrier and attenuates experimental autoimmune encephalomyelitis. J Neurochem 89:1241-1251.
Pecora AL, Rizvi N, Cohen GI, Meropol NJ, Sterman D, Marshall JL, Goldberg S, Gross P, O'Neil JD, Groene WS, Roberts MS, Rabin H, Bamat MK, Lorence RM. 2002. Phase I trial of intravenous administration of PV701, an oncolytic virus, in patients with advanced solid cancers. J Clin Oncol 20:2251-2266.
Pluchino S, Zanotti L, Rossi B, Brambilla E, Ottoboni L, Salani G, Martinello M, Cattalini A, Bergami A, Furlan R, Comi G, Constantin G, Martino G. 2005. Neurosphere-derived multipotent precursors promote neuroprotection by an immunomodulatory mechanism. Nature 436:266-271.
Stanziale SF, Petrowsky H, Adusumilli PS, Ben-Porat L, Gonen M, Fong Y. 2004. Infection with oncolytic herpes simplex virus-1 induces apoptosis in neighboring human cancer cells: a potential target to increase anticancer activity. Clin Cancer Res 10:3225-3232.
Storz P. 2005. Reactive oxygen species in tumor progression. Front Biosci 10:18811896.
Stupp R, Hegi ME, Mason WP, van den Bent MJ, Taphoorn MJ, Janzer RC, Ludwin SK, Allgeier A, Fisher B, Belanger K, Hau P, Brandes AA, Gijtenbeek J, Marosi C, Vecht CJ, Mokhtari K, Wesseling P, Villa S, Eisenhauer E, Gorlia T, Weller M, Lacombe D, Cairncross JG, Mirimanoff RO. 2009. Effects of radiotherapy with concomitant and adjuvant temozolomide versus radiotherapy alone on survival in glioblastoma in a randomised phase III study: 5-year analysis of the EORTC-NCIC trial. Lancet Oncol 10:459-466.
Stupp R, Mason WP, van den Bent MJ, Weller M, Fisher B, Taphoorn MJ, Belanger K, Brandes AA, Marosi C, Bogdahn U, Curschmann J, Janzer RC, Ludwin SK, Gorlia T, Allgeier A, Lacombe D, Cairncross JG, Eisenhauer E, Mirimanoff RO. 2005. Radiotherapy plus concomitant and adjuvant temozolomide for glioblastoma. N Engl J Med 352:987-996.
Thaci B, Ahmed AU, Ulasov IV, Tobias AL, Han Y, Aboody KS, Lesniak MS. 2012. Pharmacokinetic study of neural stem cell-based cell carrier for oncolytic virotherapy: targeted delivery of the therapeutic payload in an orthotopic brain tumor model. Cancer Gene Ther 19:431-442.
Thumma SR, Elaimy AL, Daines N, Mackay AR, Lamoreaux WT, Fairbanks RK, Demakas JJ, Cooke BS, Lee CM. 2012. Long-term survival after gamma knife radiosurgery in a case of recurrent glioblastoma multiforme: a case report and review of the literature. Case Rep Med 2012:545492.
Ulasov IV, Sonabend AM, Nandi S, Khramtsov A, Han Y, Lesniak MS. 2009. Combination of adenoviral virotherapy and temozolomide chemotherapy eradicates malignant glioma through autophagic and apoptotic cell death in vivo. Br J Cancer 100:1154-1164.
Ulasov IV, Tyler MA, Zhu ZB, Han Y, He TC, Lesniak MS. 2009. Oncolytic adenoviral vectors which employ the survivin promoter induce glioma oncolysis via a process of beclin-dependent autophagy. Int J Oncol 34:729-742.
Ulasov IV, Zhu ZB, Tyler MA, Han Y, Rivera AA, Khramtsov A, Curiel DT, Lesniak MS. 2007. Survivin-driven and fiber-modified oncolytic adenovirus exhibits potent antitumor activity in established intracranial glioma. Hum Gene Ther 18:589-602.
Yang WQ, Lun X, Palmer CA, Wilcox ME, Muzik H, Shi ZQ, Dyck R, Coffey M, Thompson B, Hamilton M, Nishikawa SG, Brasher PM, Fonseca K, George D, Rewcastle NB, Johnston RN, Stewart D, Lee PW, Senger DL, Forsyth PA. 2004. Efficacy and safety evaluation of human reovirus type 3 in immunocompetent animals: racine and nonhuman primates. Clin Cancer Res 10:8561-8576.
Yoo JY, Pradarelli J, Haseley A, Wojton J, Kaka A, Bratasz A, Alvarez-Breckenridge CA, Yu JG, Powell K, Mazar AP, Teknos TN, Chiocca EA, Glorioso JC, Old M, Kaur B. 2012. Copper chelation enhances antitumor efficacy and systemic delivery of oncolytic HSV. Clin Cancer Res 18:4931 -4941.
Zhang X, Banerjee A, Banks WA, Ercal N. 2009. N-Acetylcysteine amide protects against methamphetamine-induced oxidative stress and neurotoxicity in immortalized human brain endothelial cells. Brain Res 1275:87-95.

## Claims

1. A composition comprising a therapeutically effective amount of *N*-acetylcysteine amide (NACA) for use in a method of treating cataracts in a subject in need thereof, wherein the treatment of the cataract comprises:
a) reduction of a grade II cataract to a grade I cataract, wherein the grade is made according to Lens Opacity Classification System III; or
b) reduction of a grade III cataract to a grade II cataract, wherein the grade is made according to Lens Opacity Classification System III; or
c) reduction of a grade III cataract to a grade I cataract, wherein the grade is made according to Lens Opacity Classification System III; or
d) reduction of the cataract so there is no sign of cataract in the subject after treatment.

2. The composition for use of claim 1, wherein the composition further comprises a pharmaceutically acceptable salt or excipient.

3. The composition for use of any preceding claim, wherein the NACA is administered systemically.

4. The composition for use of claim 3, wherein the NACA is administered intraperitoneally or intravenously.

5. The composition for use of any preceding claim, wherein the NACA is administered directly onto or into the eye of the subject.

6. The composition for use of claim 5, wherein the NACA is administered intraocularly.

7. The composition for use of any preceding claim, wherein the subject is a mammal.

8. The composition for use of claim 7, wherein the mammal is a human.

9. The composition for use of any preceding claim, wherein the cataract is selected from the group consisting of nuclear sclerosis, cortical cataract and posterior subcapsular cataract.

10. The composition for use of any preceding claim, wherein the NACA is administered at between 5 and 10,000 mg/kg.

## Patentansprüche

1. Zusammensetzung, umfassend eine therapeutisch wirksame Menge von N-Acetylcysteinamid (NACA), zur Verwendung in einem Verfahren zur Behandlung von Katarakten in einem Individuum, das dessen bedarf, wobei die Behandlung des Katarakts umfasst:
a) Verringerung eines Grad-II-Katarakts zu einem Grad-I-Katarakt, wobei der Grad nach dem Lens Opacity Classification System III vergeben wird; oder
b) Verringerung eines Grad-III-Katarakts zu einem Grad-II-Katarakt, wobei der Grad nach dem Lens Opacity Classification System III vergeben wird; oder
c) Verringerung eines Grad-III-Katarakts zu einem Grad-I-Katarakt, wobei der Grad nach dem Lens Opacity Classification System III vergeben wird; oder
d) Verringerung des Katarakts, sodass nach der Behandlung kein Zeichen eines Katarakts in dem Individuum vorhanden ist.

2. Zusammensetzung zur Verwendung nach Anspruch 1, wobei die Zusammensetzung ferner ein pharmazeutisch annehmbares Salz oder einen pharmazeutisch annehmbaren Hilfsstoff enthält.

3. Zusammensetzung zur Verwendung nach einem der vorhergehenden Ansprüche, wobei das NACA systemisch verabreicht wird.

4. Zusammensetzung zur Verwendung nach Anspruch 3, wobei das NACA intraperitoneal oder intravenös verabreicht wird.

5. Zusammensetzung zur Verwendung nach einem der vorhergehenden Ansprüche, wobei das NACA direkt auf oder in das Auge des Individuums verabreicht wird.

6. Zusammensetzung zur Verwendung nach Anspruch 5, wobei das NACA intraokular verabreicht wird.

7. Zusammensetzung zur Verwendung nach einem der vorhergehenden Ansprüche, wobei es sich bei dem Individuum um ein Säugetier handelt.

8. Zusammensetzung zur Verwendung nach Anspruch 7, wobei es sich bei dem Säugetier um einen Menschen handelt.

9. Zusammensetzung zur Verwendung nach einem der vorhergehenden Ansprüche, wobei der Katarakt aus der Gruppe ausgewählt ist, die aus Kernsklerose, kortikalem Katarakt und hinterem subkapsulärem Katarakt besteht.

10. Zusammensetzung zur Verwendung nach einem der vorhergehenden Ansprüche, wobei das NACA in einer Konzentration zwischen 5 und 10,000 mg/kg verabreicht wird.

## Revendications

1. Composition comprenant une quantité thérapeutiquement efficace d'amide de N-acétylcystéine (NACA) destinée à être utilisée dans une méthode de traitement de cataractes chez un sujet qui en a besoin, le traitement de la cataracte comprenant :
a) la réduction d'une cataracte de grade II en une cataracte de grade I, le grade étant établi selon le système de classification des opacités du cristallin III ; ou
b) la réduction d'une cataracte de grade III en une cataracte de grade II, le grade étant établi selon le système de classification des opacités du cristallin III ; ou
c) la réduction d'une cataracte de grade III en une cataracte de grade I, le grade étant établi selon le système de classification des opacités du cristallin III ; ou
d) la réduction de la cataracte de sorte qu'il n'y a pas de signe de cataracte chez le sujet après traitement.

2. Composition destinée à être utilisée selon la revendication 1, la composition comprenant en outre un sel ou excipient pharmaceutiquement acceptable.

3. Composition destinée à être utilisée selon une quelconque revendication précédente, le NACA étant administré de manière systémique.

4. Composition destinée à être utilisée selon la revendication 3, le NACA étant administré par voie intrapéritonéale ou intraveineuse.

5. Composition destinée à être utilisée selon une quelconque revendication précédente, le NACA étant administré directement sur ou dans l'oeil du sujet.

6. Composition destinée à être utilisée selon la revendication 5, le NACA étant administré par voie intraoculaire.

7. Composition destinée à être utilisée selon une quelconque revendication précédente, le sujet étant un mammifère.

8. Composition destinée à être utilisée selon la revendication 7, le mammifère étant un être humain.

9. Composition destinée à être utilisée selon une quelconque revendication précédente, la cataracte étant choisie dans le groupe constitué par une sclérose nucléaire, une cataracte corticale et une cataracte sous-capsulaire postérieure.

10. Composition destinée à être utilisée selon une quelconque revendication précédente, le NACA étant administré à une hauteur comprise entre 5 et 10 000 mg/kg.
